(19)

Europäisches
Patentamt
European
Patent Office
Office européen
des brevets

(11) **EP 4 645 318 A1**

(12) **EUROPEAN PATENT APPLICATION**
published in accordance with Art. 153(4) EPC

(43) Date of publication:
05.11.2025 Bulletin 2025/45

(21) Application number: 23910483.9

(22) Date of filing: 25.12.2023

(51) International Patent Classification (IPC):
*G16B 30/00* (2019.01)

(52) Cooperative Patent Classification (CPC):
G06F 18/2135; G06F 18/24; G06Q 10/063;
G16B 30/00; G16B 40/00; G16H 70/40

(86) International application number:
PCT/CN2023/141519

(87) International publication number:
WO 2024/140557 (04.07.2024 Gazette 2024/27)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority: 26.12.2022 CN 202211677045

(71) Applicant: Tasly Stem Cell Biology Laboratory,
Tasly Group, Ltd.
Binhai New Area, Tianjin 300456 (CN)

(72) Inventors:
• KANG, Yujian James
 Tianjin 300456 (CN)
• MA, Fei
 Tianjin 300456 (CN)
• ZHANG, Jinlai
 Tianjin 300456 (CN)
• JIN, Xin
 Tianjin 300456 (CN)

(74) Representative: f & e patent
Braunsberger Feld 29
51429 Bergisch Gladbach (DE)

(54) **CELL QUALITY ATTRIBUTE TEST METHOD, TEST DEVICE AND USE**

(57) Provided are a cell quality attribute test method, a test device, and the use. The test method comprises a step of testing cell heterogeneity, the cell heterogeneity comprising the state diversity of cell subpopulations, and the state diversity of the cell subpopulations comprising any one or a combination of at least two of genome diversity, transcriptome diversity, proteome diversity, metabolome diversity and epigenome diversity of the cell subpopulations. Compared with common cell quality evaluation indices at present, taking the heterogeneity generated when cells are affected by microenvironments as a cell quality attribute can evaluate the cell product quality on the single-cell level more accurately. Accordingly, the cell quality attribute test method established on this basis has important effects in accurately testing cell product quality and ensuring clinical treatment effect stability.

Figure 1

**EP 4 645 318 A1**

## Description

### Cross reference to related applications

The present application claims priority to Chinese Patent Application No. 202211677045.3 filed on December 26, 2022, entitled "Cell quality attribute test method, test device and use", and the entire disclosure of which is incorporated herein by reference in its entirety.

### Technical Field

The present application relates to the field of cells, and more specifically, to a method, a device, and a use for detecting cell quality attributes.

### Background Art

In recent years, with the continuous development of basic theories, technical means and clinical medical exploration and research in stem cell therapy, immune cell therapy and gene editing, cell therapy products have provided new treatment opportunities for some serious and intractable diseases, and clinical studies have been conducted in various indications fields. The number of clinical studies on cell therapy products is constantly rising.

Cell therapy products have the characteristics of complex sources and compositions, diverse cell types, the ability of cells to survive in vivo, autonomously proliferate and/or differentiate, significant differences in product production scale and production process, and complex product mechanism of action. The risks of cell therapy products largely depend on the source, type, property, function, production process, non-cellular component, non-target cell population, prevention and control of contamination and/or cross-contamination throughout the production process, specific treatment approach and use and the like. Therefore, it is necessary to determine risk characteristics and special control measures based on their particular types, and adopt appropriate special technologies.

Due to factors such as complex cell sources, autonomous cell proliferation and/or differentiation, and significant differences in production processes, the cell subpopulations that make up cell products have inconsistent states, which makes the cell products heterogeneous, significantly affects the quality of cell products, and even leads to instability in clinical treatment effects.

Existing technologies cannot yet meet the needs of standardizing and guiding the research and development, registration and application of cell therapy products, and promoting industrial development. Therefore, there is a need in the art for a more accurate indicator of cell quality attributes to establish an improved analytical method for cell heterogeneity.

### Summary

In view of the deficiencies in the prior art and practical needs, the present application provides a method, a device, and a use for detecting cell quality attributes. Cell heterogeneity caused by the influence of the microenvironment is used as the cell quality attribute to establish quantitative detection methods and detection devices for cell heterogeneity. These methods and devices are applied to the preparation of cell therapy products and pharmaceutical research, which helps to establish and improve the standards for cell preparation and quality control systems.

In particular, the applicant of the present application unexpectedly found that by analyzing the state differences of different cell subpopulations in cell populations from the perspectives of genome, transcriptome, proteome, metabolome, or epigenome, and based on the single-cell expression values of molecular representations and the mean expression values of molecular representations in cell populations, quantitative detection of the degree of heterogeneity of cell populations can be achieved, and thereby establishing an improved analytical method for cell heterogeneity.

In a first aspect, the present application provides a detection method for a cell quality attribute, the detection method includes a step of detecting cell heterogeneity, wherein the cell heterogeneity includes state diversity of cell subpopulations, wherein the state diversity of cell subpopulations includes any one or a combination of at least two of genome diversity, transcriptome diversity, proteome diversity, metabolome diversity and epigenome diversity of cell subpopulations.

In some embodiments, the state diversity of cell subpopulations is the genome diversity of cell subpopulations.

In some embodiments, the state diversity of cell subpopulations is the transcriptome diversity of cell subpopulations.

In some embodiments, the state diversity of cell subpopulations is the proteome diversity of cell subpopulations.

In some embodiments, the state diversity of cell subpopulations is the metabolome diversity of cell subpopulations.

In some embodiments, the state diversity of cell subpopulations is the epigenome diversity of cell subpopulations.

The present application takes cell heterogeneity as a quality attribute and utilizes omics research methods at a single-cell level to analyze the state differences of different cell subpopulations in cell populations from the perspectives of genome, transcriptome, proteome, metabolome or epigenome, thereby achieving the effect of accurately detecting the quality of cell products.

In the present application, the genome diversity of cell subpopulations in cell populations refers to the differences in the types and/or quantities of genetic information in different cell subpopulations, which can be detected by the method of single-cell genome sequencing. The transcriptome diversity of cell subpopulations in cell populations refers to the differences in the types and/or quantities of transcripts in different cell subpopulations, which can be detected by the method of single-cell transcriptome sequencing. The proteome diversity of cell subpopulations in cell populations refers to the differences in the types and/or quantities of proteins in different cell subpopulations, which can be detected by using single-cell proteomic methods based on antibodies or mass spectrometry. The metabolome diversity of cell subpopulations in cell populations refers to the differences in the types and/or quantities of metabolites in different cell subpopulations, which can be detected by using a single-cell metabolomic method based on MALDI imaging mass spectrometry. The epigenome diversity of cell subpopulations in cell populations refers to the differences in methylation sites of different cell subpopulations, which can be detected by the method of single-cell epigenetic sequencing.

In some embodiments, the detection method includes a step of detecting the degree of cell heterogeneity, and the step of detecting the degree of cell heterogeneity includes:

determining a molecular representation and a single-cell expression value of the molecular representation based on the single-cell molecular expression matrix of cell populations;
determining a mean expression value of the molecular representation in cell populations; and
determining a degree of cell heterogeneity based on the single-cell expression value of the molecular representation and the mean expression value of the molecular representation in cell populations.

In the present application, the degree of cell heterogeneity is used to quantitatively describe the state diversity of cell subpopulations in cell populations. The molecular representation that can represent the single-cell molecular expression situation is determined based on the single-cell molecular expression matrix of cell populations, and the degree of cell heterogeneity is calculated based on the single-cell expression value of the molecular representation and the mean expression value of the molecular representation in cell populations. This helps to obtain the main information that affects cell heterogeneity while reducing noise interference, achieving the effect of accurately quantifying cell heterogeneity.

In some embodiments, the method for obtaining the single-cell molecular expression matrix of cell populations includes:

detecting a molecular expression value of cell populations at a single-cell level to obtain single-cell molecular expression data of cell populations; and
preprocessing the single-cell molecular expression data of cell populations to obtain a single-cell molecular expression matrix of cell populations;
wherein, the preprocessing comprises noise correction and/or standardization.

In some embodiments, the method for determining the molecular representation includes:
processing the single-cell molecular expression matrix by using a method of data dimensionality reduction, and selecting non-linearly related data and non-biological function related data as molecular representations.

In the present application, the original single-cell molecular expression matrix data is transformed into linearly independent data and biological function-independent data by commonly used methods for data dimensionality reduction (such as principal component analysis (PCA) and uniform manifold approximation and projection (UMAP)), thereby achieving dimensionality reduction of high-dimensional data.

In some embodiments, the molecular representation is selected from any one or a combination of at least two of gene representation, transcriptional representation, protein representation, metabolite representation, and epigenetic site representation that can represent single-cell molecular expressions of cell populations.

In some embodiments, a Euclidean distance is calculated based on the single-cell expression value of the molecular representation and the mean expression value of the molecular representation in cell populations, and the degree of cell heterogeneity is determined.

In the present application, the Euclidean distance between any two cells in the multidimensional space cell population is calculated to characterize the heterogeneity of cell populations by using the single-cell expression value of the molecular representation and the mean expression value of the molecular representation in cell populations.

In some embodiments, the degree of cell heterogeneity is determined based on the calculation formula for the degree of cell heterogeneity according to the single cell expression value of the molecular representation and the mean expression value of the molecular representation in cell populations;

the calculation formula for the degree of cell heterogeneity is as follows:

$$\text{the degree of cell heterogeneity} = \frac{200}{\pi}\tan^{-1}\left(\frac{1}{m}\sum_{i=1}^{m}\sqrt{\sum_{j=1}^{n}(X_{ij}-\mu_j)^2}+1\right)$$

wherein $X_{ij}$ represents the single-cell expression value of the j-th molecular representation in the i-th cell, n represents the number of molecular representations, m represents the cell number in cell populations, and $\mu_j$ represents the mean expression value of the j-th molecular representation in cell populations.

In the present application, the degree of cell heterogeneity is ranging from [0, 100). The degree of cell heterogeneity in cell populations is 0 when all cells in cell populations are derived from the same clone. For example, induced pluripotent stem cell (iPSC) products with no heterogeneity and uniform composition. The degree of cell heterogeneity in cell populations is infinite when each cell in cell populations is derived from a clone that is different from other cells. However, in order to better apply the degree of cell heterogeneity to the evaluation of the safety and efficacy of cell products, the maximum value of the degree of cell heterogeneity is limited to be infinitely close to 100.

In some embodiments, the detection method further includes a step of detecting any one or a combination of at least two of microbiological safety, cell markers, cell viability, and biological efficacy.

In the present application, detecting microbiological safety includes a step of detecting mycoplasma, bacteria, fungi, endotoxin, mycobacteria and the like in cell samples; detecting cell markers includes a step of detecting cell surface markers (such as CD73, CD90, CD105, CD11b, CD19, CD34, CD45 and HLA-DR); detecting cell viability includes a step of detecting cell viability, cell doubling time, cell cycle, cell apoptosis, clone formation rate and the like; detecting biological efficacy includes a step of detecting the ability of such as osteogenic differentiation, adipogenic differentiation, chondrogenic differentiation.

As a preferred technical solution, the present application provides a detection method for a cell quality attribute, the detection method includes a step of detecting cell heterogeneity, and further includes a step of detecting any one or a combination of at least two of microbiological safety, cell markers, cell viability, and biological efficacy;

wherein the step of detecting cell heterogeneity includes a step of detecting the degree of cell heterogeneity, including:

detecting whole genome/exome of cell populations at a single-cell level to obtain single-cell whole genome/exome expression data of cell populations by using a method of single-cell genome sequencing;
preprocessing the single-cell whole genome/exome expression data of cell populations to obtain a single-cell whole genome/exome expression matrix of cell populations;
determining a gene/exon representation and a single-cell expression value of the gene/exon representation based on the single-cell whole genome/exome expression matrix of cell populations;
determining a mean expression value of the gene/exon representation in cell populations; and
determining the degree of cell heterogeneity based on the calculation formula of the degree of cell heterogeneity according to the single-cell expression value of the gene/exon representation and the mean expression value of the gene/exon representation in cell populations,
the calculation formula for the degree of cell heterogeneity is as follows:

$$\text{the degree of cell heterogeneity} = \frac{200}{\pi}\tan^{-1}\left(\frac{1}{m}\sum_{i=1}^{m}\sqrt{\sum_{j=1}^{n}(X_{ij}-\mu_j)^2}+1\right)$$

wherein $X_{ij}$ represents the single-cell expression value of the j-th gene/exon representation in the i-th cell, n represents the number of gene/exon representations, m represents the cell number in cell populations, and $\mu_j$ represents the mean expression value of the j-th gene/exon representation in cell populations.

As a preferred technical solution, the present application provides a detection method for a cell quality attribute, the detection method includes a step of detecting cell heterogeneity, and further includes a step of detecting any one or a combination of at least two of microbiological safety, cell markers, cell viability, and biological efficacy;

wherein the step of detecting cell heterogeneity includes a step of detecting the degree of cell heterogeneity, including:

detecting transcript expression values of cell populations at a single-cell level to obtain single-cell gene expression data of cell populations by using a method of single-cell transcriptome sequencing;

preprocessing the single-cell gene expression data of cell populations to obtain a single-cell gene expression matrix of cell populations;

determining a transcriptional representation and a single-cell expression value of the transcriptional representation based on the single-cell gene expression matrix of cell populations;

determining a mean expression value of the transcriptional representation in cell populations; and

determining the degree of cell heterogeneity based on the calculation formula of the degree of cell heterogeneity according to the single-cell expression value of the transcriptional representation and the mean expression value of the transcriptional representation in cell population;

the calculation formula for the degree of cell heterogeneity is as follows:

$$\text{the degree of cell heterogeneity} = \frac{200}{\pi}\tan^{-1}\left(\frac{1}{m}\sum_{i=1}^{m}\sqrt{\sum_{j=1}^{n}(X_{ij}-\mu_j)^2 + 1}\right)$$

wherein $X_{ij}$ represents the single-cell expression value of the j-th transcriptional representation in the i-th cell, n represents the number of transcriptional representations, and m represents the cell number in cell populations, and $\mu_j$ represents the mean expression value of the j-th transcriptional representation in cell populations.

In a second aspect, the present application provides a detection device of a cell quality attribute, and the detection device comprises a detection module for cell heterogeneity. The detection module for cell heterogeneity is configured to detect the state diversity of cell subpopulations, wherein the state diversity of cell subpopulations includes any one or a combination of at least two of the genome diversity, transcriptome diversity, proteome diversity, metabolome diversity, and epigenome diversity of cell subpopulations.

In some embodiments, the state diversity of cell subpopulations is the genome diversity of cell subpopulations.

In some embodiments, the state diversity of cell subpopulations is the transcriptome diversity of cell subpopulations.

In some embodiments, the state diversity of cell subpopulations is the proteome diversity of cell subpopulations.

In some embodiments, the state diversity of cell subpopulations is the metabolome diversity of cell subpopulations.

In some embodiments, the state diversity of cell subpopulations is the epigenome diversity of cell subpopulations.

In some embodiments, the detection module for cell heterogeneity includes a detection submodule for the degree of cell heterogeneity, including:

a determination unit for molecular representation, for determining a molecular representation, a single-cell expression value of the molecular representation, and a mean expression value of the molecular representation in cell populations, based on a single-cell molecular expression matrix of cell populations; and

a calculation unit, for determining a degree of cell heterogeneity based on the single-cell expression value of the molecular representation and the mean expression value of the molecular representation in cell populations.

In some embodiments, the detection module for cell heterogeneity further includes a detection submodule for single-cell molecular expression matrix, including:

a detection unit for single-cell molecular expression data, for detecting a molecular expression value of cell populations at a single-cell level to obtain a single-cell molecular expression data of cell populations; and

a data preprocessing unit, for preprocessing the single-cell molecular expression data of cell populations to obtain a single-cell molecular expression matrix of cell populations; wherein the preprocessing comprises noise correction and/or standardization.

In some embodiments, the determination unit for molecular representation is used to process the single-cell molecular expression matrix by using a method of data dimensionality reduction, and select non-linearly related data and non-biological function-related data as molecular representations.

In some embodiments, the molecular representation is selected from any one or a combination of at least two of gene representation, transcriptional representation, protein representation, metabolite representation, and epigenetic site representation that can represent a single-cell molecular expression matrix of cell populations.

In some embodiments, the calculation unit is used to calculate the Euclidean distance and determine the degree of cell heterogeneity based on the single-cell expression value of the molecular representation and the mean expression value of the molecular representation in cell populations.

In some embodiments, the calculation unit is used to determine the degree of cell heterogeneity based on the calculation formula of the degree of cell heterogeneity according to the single cell expression value of the molecular representation and the mean expression value of the molecular representation in cell populations;

the calculation formula for the degree of cell heterogeneity is as follows:

$$\text{the degree of cell heterogeneity} = \frac{200}{\pi}\tan^{-1}\left(\frac{1}{m}\sum_{i=1}^{m}\sqrt{\sum_{j=1}^{n}(X_{ij}-\mu_j)^2}+1\right)$$

wherein $X_{ij}$ represents the single-cell expression value of the j-th molecular representation in the i-th cell, n represents the number of molecular representations, m represents the cell number in cell populations, and $\mu_j$ represents the mean expression value of the j-th molecular representation in cell populations.

In some embodiments, the detection device further includes: any one or a combination of at least two of a detection module for microbiological safety, a detection module for cell marker, a detection module for cell viability, and a detection module for biological efficacy.

In some embodiments, the detection device includes: a detection module for cell heterogeneity, a detection module for microbiological safety, a detection module for cell marker, a detection module for cell viability, and a detection module for biological efficacy as described above.

In a third aspect, the present application provides an application or use of the aforementioned detection method of a cell quality attribute and/or detection device of a cell quality attribute in preparing cell products.

In some embodiments, the cell product includes living mammalian cells that have been or have not been genetically modified, and any one or a combination of at least two of an optional pharmaceutically acceptable adjuvant, a carrier, an excipient, and a diluent;

wherein the mammal includes human.

In some embodiments, the cell product includes any one or a combination of at least two of autologous or allogeneic stem cell products, stromal cell products, immune cell products, tissue cell products, and cell line products.

In some embodiments, the stem cell includes any one or a combination of at least two of an adult stem cell, an embryonic stem cell, an induced pluripotent stem cell, a stem cell derived from the transformation of a mature somatic cell, and cells derived therefrom.

In some embodiments, the stem cell includes any one or a combination of at least two of a mesenchymal stem cell, a mesenchymal stromal cell, a multipotent stromal cell, a multipotent mesenchymal stromal cell, and a drug signaling cell.

In some embodiments, the stem cell includes any one or a combination of at least two of an adipose-derived stem cell, an umbilical cord-derived stem cell, a placenta-derived stem cell, a bone marrow-derived stem cell, a dental pulp-derived stem cell, a uterine blood-derived stem cell, an amniotic epithelial stem cell, and a bronchial basal layer cell.

In a fourth aspect, the present application provides an application or use of the aforementioned detection method of a cell quality attribute and/or detection device of a cell quality attribute in the research and/or development of cell products.

In some embodiments, the cell product includes a cell stock product and a cell preparation product, the cell stock product includes a cell seed bank product and a cell working bank product, and the research and/or development of cell products includes the research and/or development of the preparation process of cell seed bank products (including donor screening process, primary separation process, multiplication culture process, freezing process and the like), the storage stability of cell seed bank products, the preparation process of cell working bank products (including donor screening process, primary separation process, multiplication culture process, freezing process and the like), the storage stability of cell working bank products, the preparation process of cell preparation products (including working bank cell thawing process, cleaning process, filling process and the like) or the stability of cell preparation products (including storage stability, transportation stability, stability in use, joint stability and the like).

Compared with the prior art, the present application has the following beneficial effects:

(1) As demonstrated in embodiments, the degree of heterogeneity in cell populations is correlated with the safety and efficacy of cells. Cell heterogeneity, as a cell quality attribute, can be used to evaluate cell quality. The present application uses the heterogeneity of cells affected by the microenvironment as a cell quality attribute. Compared with the currently commonly used cell quality assessment indicators, the present application can more accurately assess the quality of cell products at the single-cell level. The detection method of a cell quality attribute established thereby plays an important role in accurately detecting the quality of cell products and ensuring the stability of clinical treatment effects.

(2) The degree of cell heterogeneity is calculated in the present application based on the single-cell expression value of the molecular representation and the mean expression value of the molecular representation in cell populations, thereby achieving the effect of quantitatively describing the state diversity of cell subpopulations in cell populations; and

(3) The detection method and detection device of a cell quality attribute of the present application have important application value in the preparation, research, and development of cell products, and provide a new evaluation means for determining the preparation process and preservation process of cell products.

## Brief Description of the Drawings

Figure 1 shows the score of cell heterogeneity degree of different cell samples S1, S2, and S3.
Figure 2A is a schematic diagram showing the main components of a detection device of a cell quality attribute.
Figure 2B is a schematic diagram showing the main components of the detection module 10 for cell heterogeneity.
Figure 2C is a schematic diagram showing the main components of a detection submodule 110 for single-cell molecular expression matrix.
Figure 2D is a schematic diagram showing the main components of a detection submodule 120 for cell heterogeneity. The reference numerals are as follows:
10 - detection module for cell heterogeneity, 20 - detection module for microbiological safety, 30 - detection module for cell marker, 40 - detection module for cell viability, 50 - detection module for biological efficacy; wherein, 110 - detection submodule for the matrix of single cell molecular expression, 120 - detection submodule for the degree of cell heterogeneity; wherein, 1101 - detection unit for single - cell molecular expression data, 1102 - data preprocessing unit; wherein, 1201 - determination unit for molecular representation, 1202 - calculation unit.

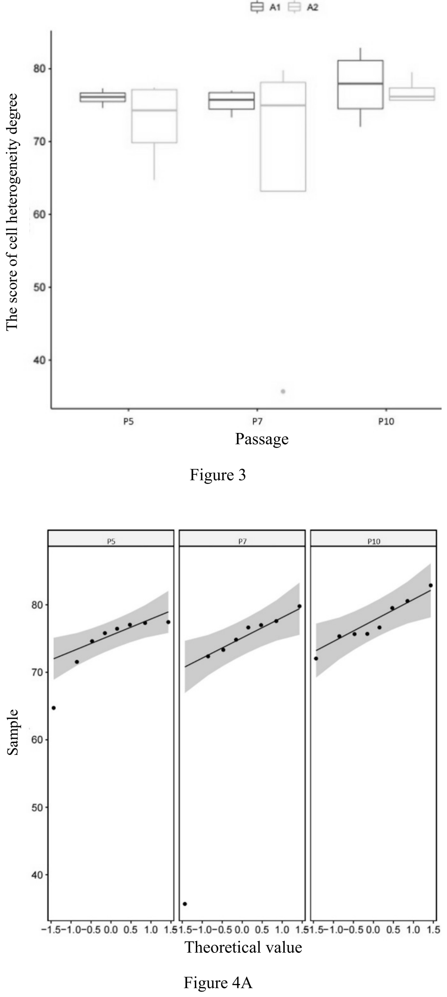

Figure 3 shows the score distribution of cell heterogeneity degree for P5, P7 and P10 ADSCs samples in two batches (A1 and A2).

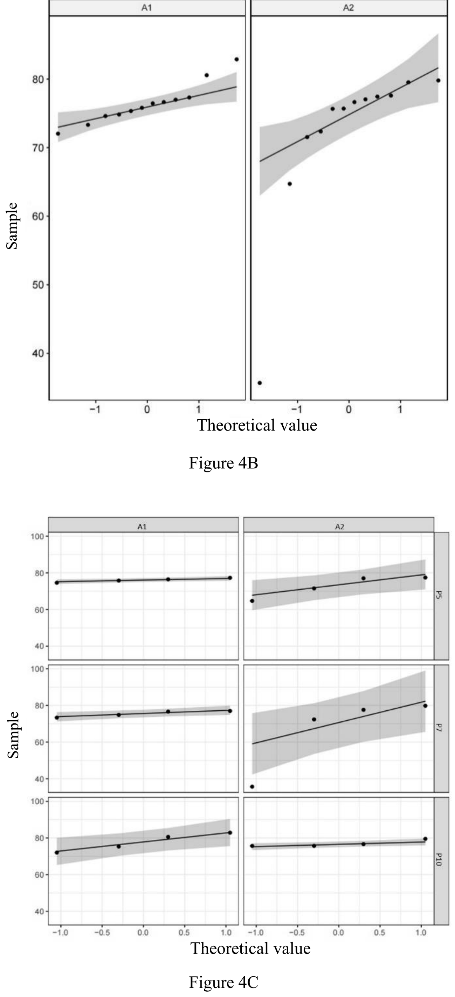

Figure 4A is a Q-Q normality test of the distribution of cell heterogeneity of ADSCs samples in different generations, Figure 4B is a Q-Q normality test of the cell heterogeneity of ADSCs samples in different batches, and Figure 4C is a Q-Q normality test of the cell heterogeneity of ADSCs samples in multiple generations and batches.

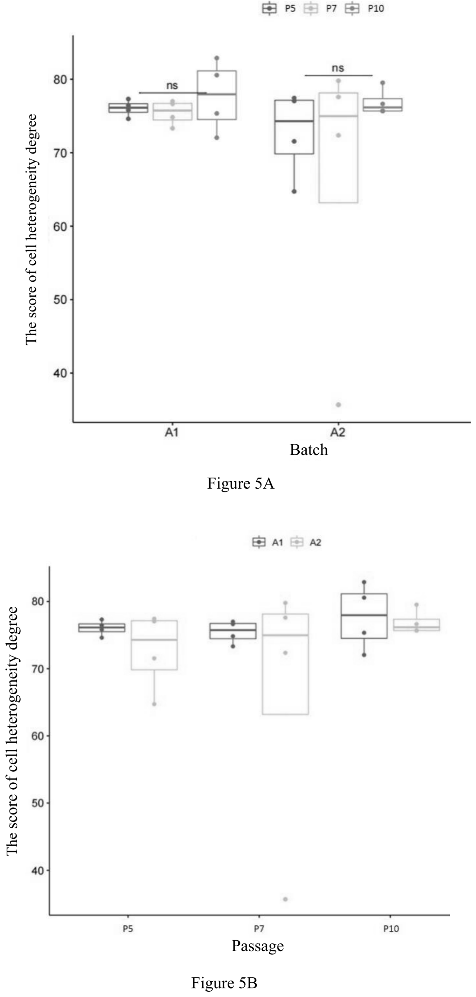

Figure 5A shows the differences in the degree of cell heterogeneity of ADSCs samples in the same batch but different generations, and Figure 5B shows the differences in the degree of cell heterogeneity of ADSCs samples in the same generation but different batches.

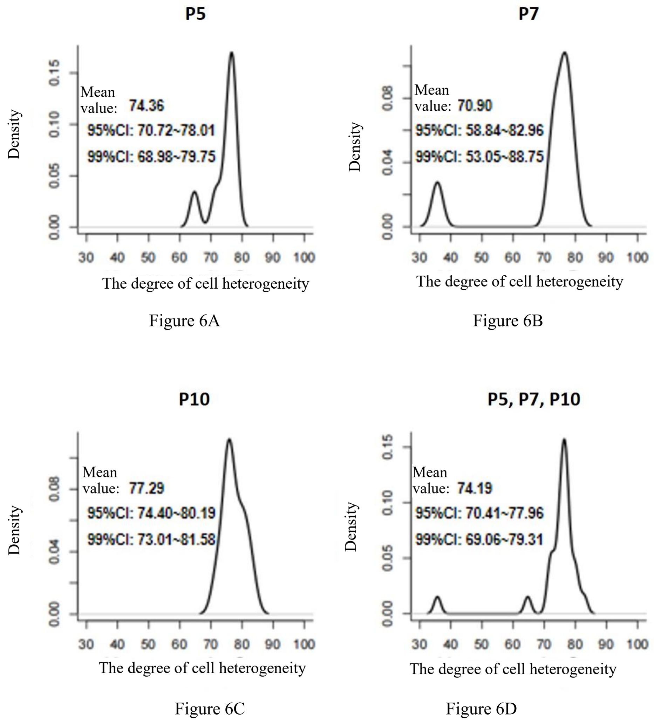

Figure 6A shows the reference value range for the cell heterogeneity score of P5 ADSCs, Figure 6B shows the reference value range for the cell heterogeneity score of P7 ADSCs, Figure 6C shows the reference value range for the cell heterogeneity score of P10 ADSCs, and Figure 6D shows the reference value range for the comprehensive cell heterogeneity score of P5, P7 and P10 ADSCs.

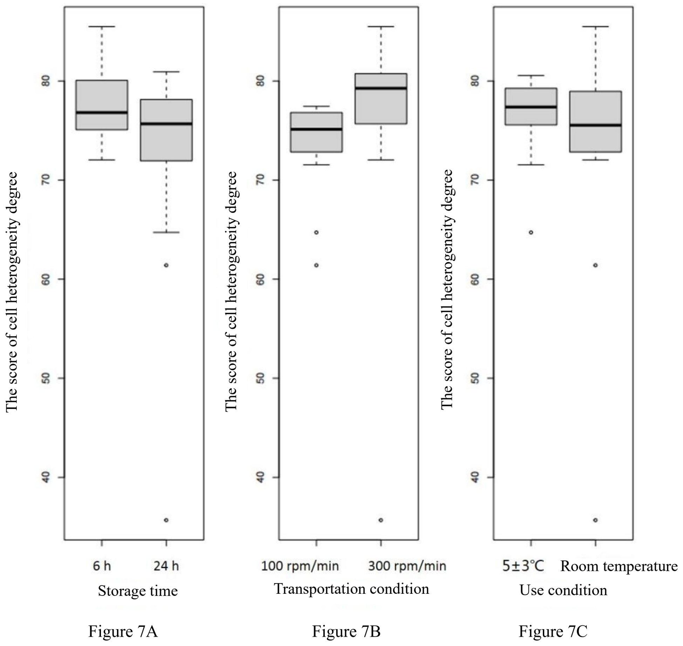

Figure 7A shows the score distribution of cell heterogeneity for ADSCs injection left at 5±3°C for 6h or 24h, Figure 7B shows the score distribution of cell heterogeneity for ADSCs injection agitated at 5±3°C at low speed (100 rpm/min) or high speed (300 rpm/min), and Figure 7C shows the score distribution of cell heterogeneity for ADSCs injection left at 5 ±3°C or room temperature (10-30°C) for 2h.

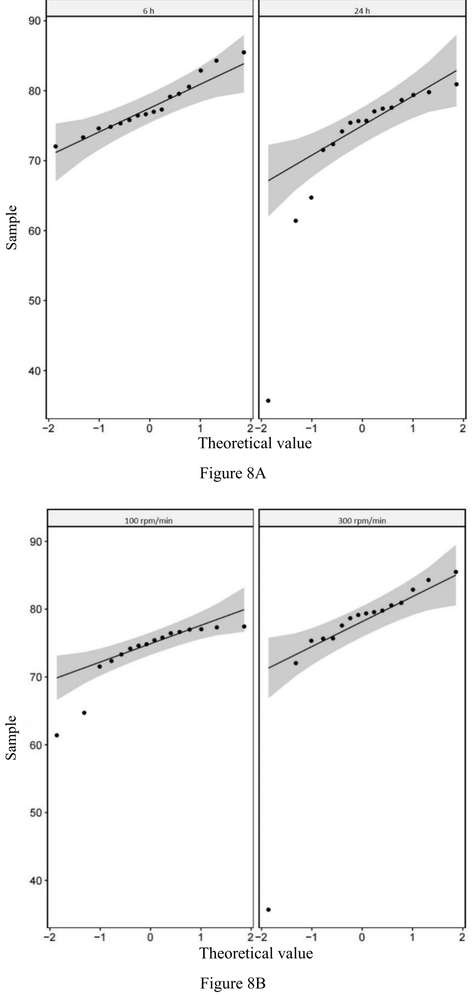

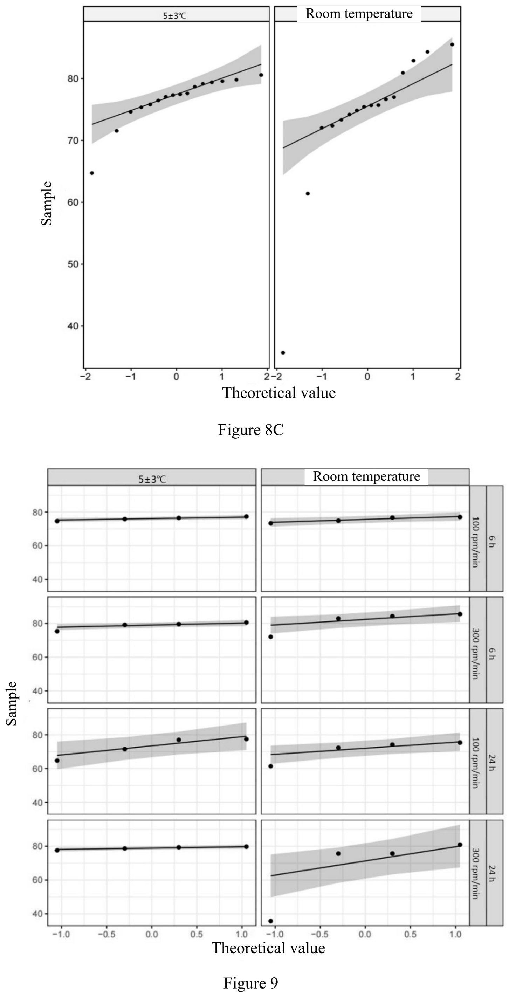

Figure 8A is a Q-Q normality test of the degree of cell heterogeneity for ADSCs injection at different storage time, Figure 8B is a Q-Q normality test of the degree of cell heterogeneity for ADSCs injection under different transportation conditions, and Figure 8C is a Q-Q normality test of the degree of cell heterogeneity for ADSCs injection under different use conditions.
Figure 9 is a Q-Q normality test of the degree of cell heterogeneity for ADSCs injection under joint influences of multiple factors.

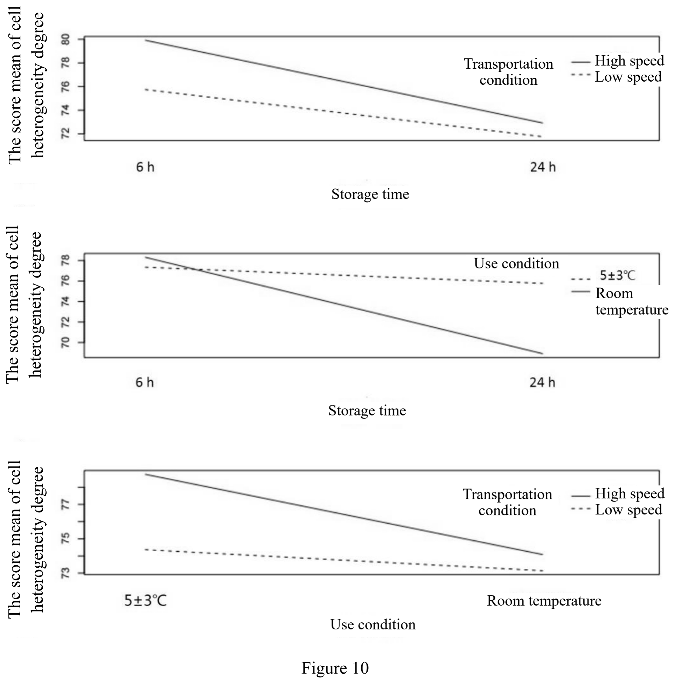

Figure 10 shows the interactive effects of storage time, transportation conditions, and use conditions on the degree of cell heterogeneity for ADSCs injection.

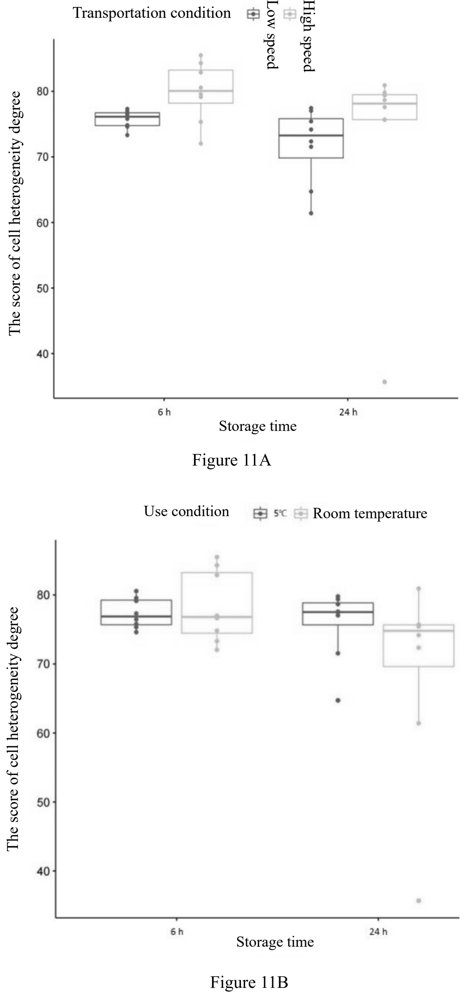

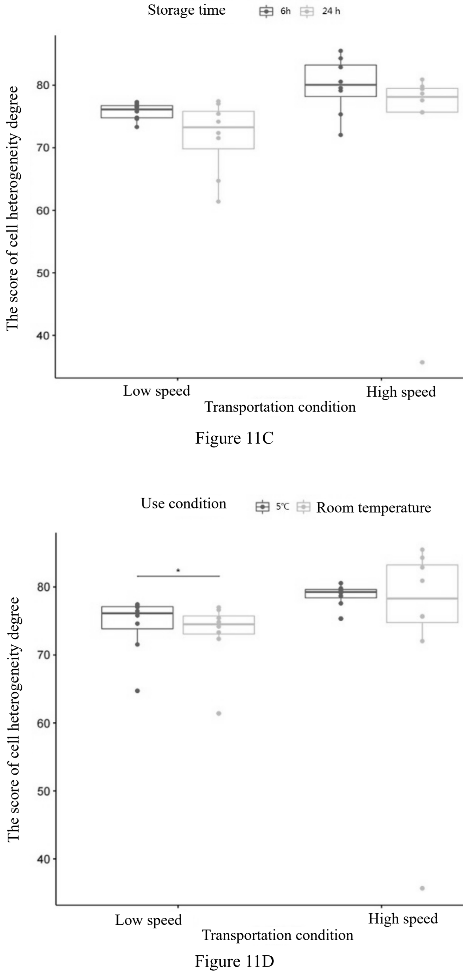

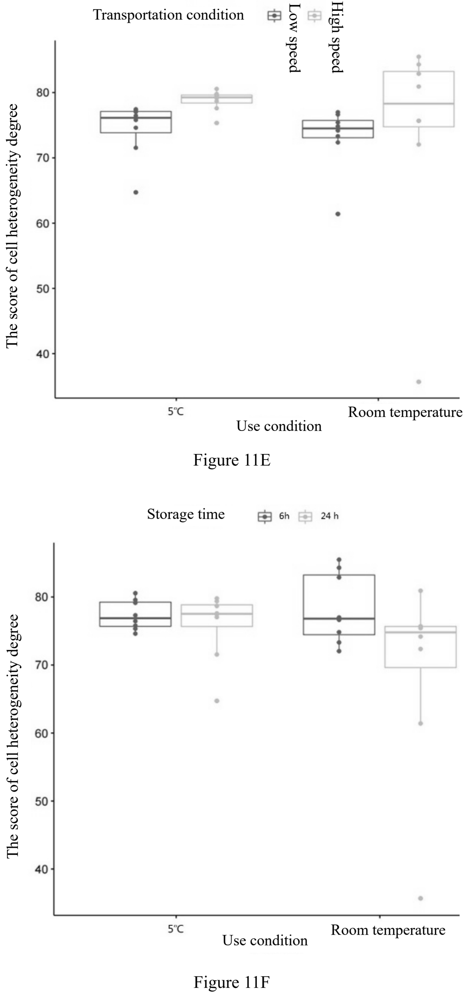

Figure 11A shows the effects of transportation conditions on the degree of cell heterogeneity for ADSCs injection under certain storage time, Figure 11B shows the effects of use conditions on the degree of cell heterogeneity for ADSCs injection under certain storage time, Figure 11C shows the effects of storage time on the degree of cell heterogeneity for ADSCs injection under certain transportation conditions, Figure 11D shows the effects of use conditions on the degree of cell heterogeneity for ADSCs injection under certain transportation conditions, Figure 11E shows the effects of transportation conditions on the degree of cell heterogeneity of ADSCs injection under certain use conditions, and Figure 11F shows the effects of storage time on the degree of cell heterogeneity of ADSCs injection under certain use conditions.

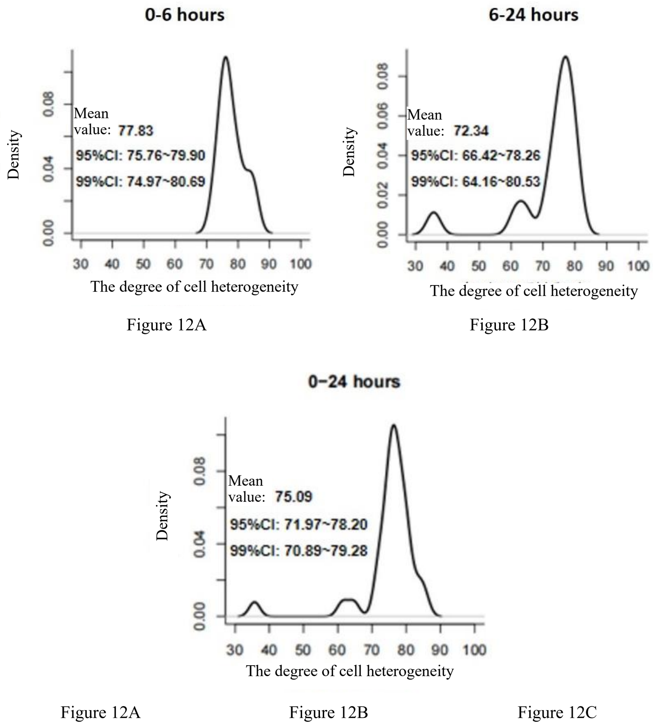

Figure 12A is the reference value range for the cell heterogeneity score for ADSCs stored for 0-6h, Figure 12B is the reference value range for the cell heterogeneity score for ADSCs stored for 6-24h, and Figure 12C is the reference value range for the cell heterogeneity score for ADSCs stored for 0-24h.

## Specific Modes for Carrying Out the Embodiments

To further illustrate the technical means and effects of the present application, the present application is further described below in conjunction with embodiments and drawings. It should be understood that the specific embodiments described herein are only used to explain the present application rather than to limit the present application. It is apparent to a person skilled in the art that various modifications and variations for the method and system of the present application can be made without departing from the scope and essence of the present application. Although the application has been described in conjunction with specific preferred embodiments, it should be understood that the present application should not be unduly limited to these specific embodiments, as claimed, and that various modifications and additions to the described embodiments may be made within the scope of the present application. Various modifications made to the described embodiments for the purpose of implementing the present application to a person skilled in the art of molecular biology and related fields are within the scope of protection of the claims.

If no specific techniques or conditions are specified in Examples, the techniques or conditions described in the literature in the art or the product manual shall be followed. The reagents or instruments used without indicating the manufacturer are all conventional products that can be commercial available through regular channels.

definition

As described in the context, a "cell quality attribute" refers to a certain physical, chemical, biological or microbiological property of cells, which should have an appropriate limit, range or distribution to ensure an expected quality of cell products.

As described in the context, a "cell population" refers to one or more cell groups of interest that have the same or different attributes and/or functions. A "cell subpopulation" refers to a cell group in a "cell population" that is further classified by differences in state, such as differences in cell morphology, cell function and/or cell molecular markers.

As described in the context, "cell heterogeneity" refers to the differences in the existence states of different cell subpopulations in cell populations. Such differences can be genome differences, transcriptome differences, proteome differences, metabolome differences, and epigenome differences, which are manifested in differences in aspects such as cell morphology, cell function, or cell molecular markers in different cell subpopulations. (Reference: Kumar RM, Cahan P, Shalek AK, Satija R, DaleyKeyser A, Li H, Zhang J, Pardee K, Gennert D, Trombetta JJ, Ferrante TC, Regev A, Daley GQ, Collins JJ. Deconstructing transcriptional heterogeneity in pluripotent stem cells. Nature. 2014 Dec 4;516(7529):56-61.)

As described in the context, the "degree of cell heterogeneity" refers to a quantitative value that describes the state diversity of cell subpopulations in cell populations.

As described in the context, a "single-cell molecular expression matrix" refers to a matrix containing molecular expression information of each cell, wherein the rows of a single-cell molecular expression matrix represent the expression of specific molecules in different cells, the columns represent the expression of different molecules by specific cells, and the data in the matrix represent the expression amount of specific molecules in specific cells.

As described in the context, a "molecular representation" refers to gene representation, transcriptional representation, protein representation, metabolite representation and epigenetic site representation that can represent single-cell molecular expression in cell populations.

As described in the context, a "Euclidean distance" refers to the actual distance between two points in a multidimensional space.

As described in the context, "cell products" refer to living cell products, including genetically modified or unmodified cells, such as autologous or allogeneic immune cells, stem cells, stromal cells, tissue cells or cell lines, also known as "cell therapy products".

## Example 1 Cell acquisition and cultivation

I. Acquisition of cells

1. Collection of adipose tissue

Adipose tissue from donors (all negative for human immunodeficiency virus (HIV), hepatitis B virus (HBV), hepatitis C virus (HCV), human T-cell lymphotropic virus (HTLV), Epstein-Barr virus (EBV), cytomegalovirus, and Treponema pallidum) was collected in a sterile environment;

    50-150 mL of adipose tissue was taken and put into a sealed container pre-added with 100 mL of tissue preservation solution (purchased from Tianjin Haoyang Biological Manufacture Co., Ltd.), and stored at a constant temperature of 2-8°C for future use;
    30 mL of tissue preservation solution was aspirated with a pipette, and a human adipose-derived cell was isolated and cultured after confirming the absence of bacteria, endotoxin and mycoplasma contamination.

2. Isolation of cells

An equal volume of Dulbecco's phosphate buffered solution (dPBS) was added to the adipose tissue, the container containing the tissue was sealed, shaken vigorously for 20 seconds and stood for 5 minutes. After completely layering the adipose tissue from dPBS, the liquid layer below the tissue was aspirated and discarded. The adipose tissue was repeatedly rinsed with dPBS until no obvious red color was observed in the liquid layer below the tissue.

20 mL of each aliquot of the washed adipose tissue was added to a 50 mL Falcon tube, and an equal volume of dPBS was added, and then centrifuged at 400 g for 5 min. The solution was divided into an upper layer of lipid, a middle layer of lipid tissue, a lower layer of dPBS, and blood cell pellet. The upper layer of lipid, bottom layer of dPBS, and blood cell pellet were removed.

Twice the volume of 1 mg/mL type I collagenase (digestion solution) was added to the adipose tissue. The container containing the tissue was sealed, transferred to a thermostatic air shaker preheated at 37°C, and digested at 120 rpm/min for 1 h.

3. Collection of cells

The digested tissue was centrifuged at 500 g for 8 min at room temperature. The solution was divided into an upper layer of lipid, a middle layer of lipid tissue, a lower layer of digestion solution, and a bottom layer of cell pellet after centrifugation. The upper layer of lipid, middle layer of lipid tissue, and lower layer of digestion solution were aspirated and discarded. The bottom layer of cell pellet was resuspended with dPBS, filtered into a 50-mL Falcon tube with a 100-$\mu$m sieve, and centrifuged at 500 g for 5 min. The supernatant was removed to obtain a cell pellet containing P0 adipose-derived stromal cells (ADSCs).

Complete medium equal in volume to that of the adipose tissue was added to a Falcon tube, and mixed evenly to allow the digested cells to be fully freed in the complete medium, thereby obtaining a cell suspension containing P0 ADSCs.

II. Cell Cultivation

ADSCs were cultured and proliferated by using culture medium (DMEM/F-12+5% Helios UltraGRO-Advanced, DMEM/F-12 is purchased from Thermo Fisher Scientific Inc., Helios UltraGRO-Advanced is purchased from Helios BioScience Inc.), and the specific steps are as follows:

1. Primary culture

The cell pellet was resuspended in a medium equal to the volume of adipose tissue, and 1.5 mL of the cell suspension was taken and inoculated into a T75 flask for cell culture pre-added with 8.5 mL of medium;

The T75 flask for cell culture was labelled and transferred to a cell culture incubator, and cultured at 37°C and 5% $CO_2$. The adipose-derived cells have basically adhered to the wall after 24 h. The supernatant was removed and 10 mL of culture medium was added. Thereafter, the medium was changed every three days.

It was observed and found through microscope that in addition to P0 ADSCs, the obtained primary cells also contained many miscellaneous cells and matrix components. ADSCs were in a typical long spindle shape.

2. Subculture

The medium was removed when a confluence of P0 cells reached 50% to 70%, 10 mL of dPBS was added and washed once, and the washing solution was removed. Then 1.5 mL of digestion solution Tryple™-Express (1×) was added to digest for 1-2 min. When most cells became round and fell off, the culture flask was gently tapped and 4.5 mL of dPBS was added to terminate the digestion.

The liquid after termination was collected in a 50-mL Falcon tube, 10 mL of dPBS was added and washed once, and centrifuged at 400 g for 5 min. The upper layer was a mixture of digestion solution and dPBS, and the lower layer of white pellet was the pellet containing P0 ADSCs. The supernatant was removed and the white pellets in multiple Falcon tubes was collected in one Falcon tube. Medium was added to resuspend cells, and was made up to 30 mL. The cell suspension was mixed by pipetting up and down, and samples were taken for cell counting;

Cells were centrifuged at 400 g for 5 min after counting, and the supernatant was removed. Medium was added to resuspend cells, and was mixed by pipetting up and down. The cell suspension was inoculated into a cell culture flask after making up to volume, so that the density of passage cells was 5000-6000 cells/cm$^2$;

The cell culture flask was labeled with information such as cell batch, passage number, and culture time, and cultured in a cell culture incubator. Cells were passaged again when a cell confluence reached about 90%, and P5 ADSCs was collected and cryopreserved to establish a working cell bank.

According to the preceding steps in the present Example, three working cell banks were recovered as P5 ADSCs samples, which were named S1, S2 and S3, respectively.

## Example 2 Routine Quality Control

According to the following steps in the present Example, routine quality control was performed on three samples S1, S2 and S3.

I. Microbiological safety test

1. Sterility test

The test steps are detailed in Sterility Test Method, General Principle 1101 of the Pharmacopoeia of the People's Republic of China, Edition 2020 (Part IV), which is briefly described as follows:

100 mL of 0.9% sodium chloride injection (purchased from SJZ No. 4 Pharmaceutical) was used to filter and wet the filter membrane of a disposable triple germs collector (purchased from Zhejiang Tailin Biotechnology Co., Ltd.) in advance, and then the cell sample was introduced into the germs collector for filtration. After the cell sample was filtered, the filter membrane was rinsed twice with 300 mL of 0.9% sodium chloride injection;

100 mL of fluid thioglycollate medium was added to two culture vessels in the triple germs collector containing cell samples, and 100 mL of Tryptic Soy Broth was added to the other culture vessel;

0.9% sodium chloride injection was used to instead a cell sample as a negative control, and *Staphylococcus aureus* (the inoculation amount of bacteria was less than 100 CFU) was used as a positive control;

each experimental group was gently shake after inoculation. One culture vessel containing fluid thioglycollate medium was selected and cultured at 30-35°C for each experimental group, and the remaining culture vessels are placed at 20-25°C for culturing. The culture is carried out for a total of 14 days. The growth of bacteria was observed and recorded every working day during the culturing.

2. Mycoplasma test

The test steps are detailed in Mycoplasma Test Method, General Rule 3301 of the Pharmacopoeia of the People's Republic of China, Edition 2020 (Part IV), which is briefly described as follows:

mycoplasma broth medium, mycoplasma broth medium containing arginine, mycoplasma semi-fluid medium and mycoplasma semi-fluid medium containing arginine were prepared and sterilized according to conventional formulas. 800,000 IU of sodium penicillin for injection (purchased from Jiangxi Dongfeng Pharmaceutical Co., Ltd.) were re-dissolved with 1 mL of 0.9% sodium chloride injection for future use. 200 mL of fetal bovine serum and 800,000 IU of sodium penicillin for injection were added to every 800 mL of sterilized medium, mixed well and stored at 2-8°C.

4 tubes of mycoplasma broth medium (10 mL each), 4 tubes of mycoplasma broth medium containing arginine, 2 tubes of mycoplasma semi-fluid medium, and 2 tubes of mycoplasma semi-fluid medium containing arginine with a capacity of 10 mL each were taken, inoculated with 1.0 mL of cell samples, cultured at 36°C±1°C for 21 days, and observed every 3 days;

on the 7th day after inoculation, 2 tubes of mycoplasma broth medium inoculated with cell samples and 2 tubes of mycoplasma broth medium containing arginine inoculated with cell samples for subculturing; each tube of mycoplasma broth medium is transferred to 2 tubes of mycoplasma semi-fluid medium and 2 tubes of mycoplasma broth medium, and each tube of mycoplasma broth medium containing arginine is transferred to 2 tubes of mycoplasma semi-fluid medium containing arginine and 2 tubes of mycoplasma broth medium containing arginine. The inoculation volume of the medium of each tube was 1 mL. The resultants were placed and cultured at 36°C±1°C for 21 days and observed every 3-5 days.

3. Endotoxin test

The test steps are detailed in Bacterial Endotoxin Test Method, General Rule 1143 of the Pharmacopoeia of the People's Republic of China, Edition 2020 (Part IV), which is briefly described as follows:

the endotoxin working standard (purchased from Zhanjiang A&C Biological Co., Ltd.) was re-dissolved with 1 mL of water for endotoxin test (purchased from Zhanjiang A&C Biological Co., Ltd.), and gradient diluted after mixing by a vortex oscillator for 15 min. The solution was mixed by a vortex oscillator for 30 s in each dilution step, and finally diluted into standard solutions with $4\lambda$ and $2\lambda$ endotoxin;
the cell cryopreservation solution was placed in a water bath at 37°C for rapid thawing, and centrifuged at 1200 rpm for 5 min. The supernatant was taken and added with endotoxin test for water for gradient dilution. The solution was mixed by a vortex oscillator for 30 s in each dilution step. The dilution ratio did not exceed the maximum valid dilution (MVD), and the dilution was used as a test solution for cell samples. The maximum valid dilution (MVD) was calculated according to the formula $MVD=C*L/\lambda$, wherein L was the endotoxin limit of the cell sample, C was the concentration of the test solution for cell sample, and $\lambda$ was the labeled sensitivity of Limulus reagent;
another test solution for cell samples was taken and added with standard solution of $4\lambda$ endotoxin at a volume ratio of 1:1, and mixed by a vortex oscillator for 30 s to serve as the endotoxin positive control solution for cell samples;
8 tubes of Limulus reagents (purchased from Zhanjiang A&C Biological Co., Ltd.) were each re-dissolved with 0.1 mL of endotoxin test for water; 0.1 mL of endotoxin positive control solution for cell samples was added to 2 tubes of Limulus reagents to serve as positive product control (PPC) for cell samples in parallel; 0.1 mL of standard solution with $2\lambda$, endotoxin was added to 2 tubes of Limulus reagents to serve as positive control (PC) in parallel; 0.1 mL of endotoxin test for water was added to 2 tubes of Limulus reagents to serve as negative control (NC) in parallel; 0.1 mL of test solution for cell samples with a dilution factor not exceeding MVD was added to 2 tubes of Limulus reagents to serve as test tubes for cell sample in parallel; and
the reaction hole cover of the preheated gel method analyzer for bacterial endotoxin was opened, a reaction tube was put in, and the countdown for 60 minutes was started. The reaction tube was taken out 1 minute before the end of 60 minutes, and the results were observed and recorded.

The results were shown in Table 1. After 14 days, there was no bacterial growth in the germs collector containing cell samples, indicating the sterility test results for S1, S2, and S3 were eligible; the mycoplasma test for S1, S2, and S3 was negative, indicating the test results were eligible; and the endotoxin test for S1, S2, and S3 was negative, indicating the test results were eligible.

Table 1

| Sample name | Sterility test | Mycoplasma test | Endotoxin test |
|---|---|---|---|
| S1 | Negative | Negative | Negative |
| S2 | Negative | Negative | Negative |
| S3 | Negative | Negative | Negative |

II. Cell marker detection

The expression of mesenchymal stem cell-specific surface markers CD73, CD90, CD105, CD11b, CD19, CD34, CD45, and HLA-DR on cell samples was detected (see reference: M. Dominici et al., Minimal criteria for defining multipotent mesenchymal stromal cells. The International Society for Cellular Therapy position statement. Cytotherapy (2006) Vol. 8, No. 4, 315-317), and the steps were as follows:
Tryple™-Express (1×) was added into cell samples in good growth condition, and they were digested at 37°C for 2-3 min. A volume of PBS (1×) which is more than 3 times the volume of Tryple™-Express (1×) was added to terminate the digestion. Cells were rinsed slowly, so as to detach and loosen into individual cells; the cell suspension was aspirated into a 50 mL Falcon tube, and centrifuged at 300 g for 5 min; the supernatant was discarded, 1×PBS was added to resuspend cells, and centrifuged at 300 g for 5 min; the supernatant was discarded, 1×PBS was added to resuspend cells for future use.
The cell concentration was adjusted to a density of live cells at $(0.5-1)\times10^7$ cells/mL, 100 µL of the cell suspension was aspirated into a flow tube, and then 5 µL of FITC-labeled anti-human CD34 antibody, FITC-labeled anti-human CD45 antibody, FITC-labeled anti-human CD11b antibody, FITC-labeled anti-human HLA-DR antibody, FITC-labeled anti-human CD73 antibody, FITC-labeled anti-human CD90 antibody, APC-labeled anti-human CD19 antibody and PE-labeled anti-CD105 antibody were each added. FITC-labeled mouse IgG1, APC-labeled mouse IgG1, and PE-labeled mouse IgG1 were each added as control groups. The cell suspension was mixed with antibodies by vortex oscillation, and incubated for 15 min in the dark.

2 mL of sheath fluid was added into each tube, vortexed, and centrifuged at 300 g for 5 min, to discard the supernatant. Cells were resuspended with 300 μL of PBS buffer containing 1% paraformaldehyde, and detected by using a flow cytometry.

The results showed that the expression rates of S1, S2, and S3 for positive surface markers of mesenchymal stem cells CD73, CD90, and CD105 were higher than 95%, and the expression rates of negative markers of mesenchymal stem cells CD11b, CD19, CD34, CD45, and HLA-DR were lower than 2%.

III. Cell Viability Assay

1. Cell viability assay

The cell sample was diluted with 0.9% sodium chloride injection and mixed thoroughly with 0.4% trypan blue dye at a volume ratio of 9:1; 10 μL of mixture was aspirated into the counting chamber of a disposable counting plate and observed under a 10× objective lens. The total number of live cells and dead cells in four squares were recorded, respectively. The cell viability was calculated according to the following formula.

cell viability (%) = total number of live cells / (total number of live cells + total number of dead cells) × 100%

The results showed that the cell viability of S1, S2, and S3 all reached over 80%.

IV. Biological efficacy analysis

1. Adipogenic differentiation assay

Before the experiment, solution A and solution B were prepared according to the manual of the OriCell™ human adipose-derived mesenchymal stem cell adipogenic differentiation kit (purchased from Cyagen Biotechnology Co., Ltd.), and then the following steps were performed:

cells were seeded in a 6-well plate at a cell density of $2\times10^4$ cells/cm$^2$ with 2 mL of complete medium per well. Cells were incubated at 37°C and 5% $CO_2$ until cells were 100% confluence;
the spent growth medium was aspirated and discarded, and 2 mL of solution A was added per well. 3 days after induction, solution A was replaced with 2 mL of solution B, and then solution A was replaced after 24 hours. After repeating the replacement of solutions A and B 3 times, cells were incubated continuously with solution B to maintain for 4-7 days until the lipid droplets became large and round enough. During the incubation period of solution B for maintenance, replace with fresh solution B every 2-3 days.

Oil Red O staining was used for analysis and red lipid droplets were observed under the microscope.

2. Osteogenic differentiation assay

Before the experiment, the induction medium was prepared according to the manual of the OriCell™ human adipose-derived mesenchymal stem cells osteogenic differentiation kit (purchased from Cyagen Biotechnology Co., Ltd.), and then the following steps were performed:

cells were seeded in a 6-well plate at a cell density of $2\times10^4$ cells/cm$^2$ with 2 mL of complete medium per well. Cells were incubated at 37°C and 5% $CO_2$ until cells were 80-90% confluence;
the spent growth medium was aspirated and discarded, and 2 mL of induction medium was added per well. The medium was changed every 3 days. After 2-4 weeks of induction, the morphological changes and growth of the cells were observed. Alizarin red was used for staining to analyze and observe the red calcium nodules under a microscope.

3. Chondrogenic differentiation assay

Before the experiment, a complete chondrogenic differentiation medium was prepared according to the manual of the OriCell™ human adipose-derived mesenchymal stem cell chondrogenic differentiation kit (purchased from Cyagen Biotechnology Co., Ltd.), and then the following steps were performed:
0.1% gelatin was added to a 6-well culture plate, shaken gently until the bottom of the wells was covered, and let stand for 30 min. The gelatin was discarded, and the culture plate was dried; cells in good growth condition were seeded in a 6-well plate at a cell density of $1 \times 10^4$ cells/cm$^2$ with 2 mL of complete medium per well. Cells were placed and incubated at 37°C and 5% $CO_2$ until cells were 80-90% confluence; the culture supernatant was aspirated off from induction wells. Each well was replaced with 2 mL of freshly complete chondrogenic differentiation medium and 20 μL of TGF-β3 every 2-3 days. Cells were incubated continuously for induction at the condition of 37°C and 5% $CO_2$; control wells were incubated continuously with a complete medium. After continuous induction for more than 14 days, the cells were fixed and stained with Alcian blue, and the effect of Alcian blue staining was observed under a microscope.

The results showed that S1, S2, and S3 were induced to undergo adipogenic differentiation, osteogenic differentiation, and chondrogenic differentiation in the in vitro culture environment. From the results of Oil Red O staining, Alizarin Red staining, and Alecin Blue staining, it can be seen that S1, S2, and S3 were successfully induced into adipogenic cells, osteogenic cells, and chondrogenic cells, respectively.

**Example 3 Single-cell RNA sequencing**

In order to explore the heterogeneity of cells during culturing, single-cell RNA sequencing technology was used to detect the transcriptome profiles of S1, S2, and S3 at the single-cell level in the present Example. The steps were as follows:

I. Preparation of single-cell suspension

S1, S2, and S3 in the logarithmic growth phase were diluted with sample buffer to a cell suspension with a concentration of <1000 cells/μL; 1 μL Calcein AM dye and 1 μL Draq7 dye were added to 200 μL of cell suspension for cell staining;

the stained cell suspension was filtered with a 40-μm filter membrane. It was added to a cell chamber and placed in a cell scanner (purchased from BD Rhapsody Scanning) to calculate the cell concentration and cell viability;
the cell suspension was diluted according to the cell concentration and cell loading amount.

II. Single-cell sorting

The diluted cell suspension was added into the BD Cartridge plate for single-cell sorting (purchased from BD Biosciences, Cat: 633733), and loaded into a cell scanner. The cell loading amount and doublet rate were analyzed to evaluate the separation effect of single cells;

the unloaded cell suspension was washed with buffer. The capture beads were added to the BD Cartridge plate for single cell sorting, and loaded into a cell scanner. The bead & cell loading amount and doublet rate were analyzed to evaluate the number of capture beads bound to wells of single cells;
the excess capture beads were washed. The cell lysate was added to the BD Cartridge plate for single-cell sorting to lyse cells so that the mRNA is connected to a probe on the surface of capture beads; capture beads were recovered into a Falcon tube from the BD Cartridge plate for single-cell sorting.

III. Single-cell cDNA synthesis and library construction

Kits purchased from BD Biosciences were used for single-cell cDNA synthesis and library construction, specifically a single-cell cDNA synthesis kit (purchased from BD Biosciences, Cat: 633731), and a library construction kit (purchased from BD Biosciences, Cat: 633801). The following steps were performed according to the manual in the kit, which was briefly described as follows:
the recovered capture beads were washed, and reverse transcription reagent (Table 2) was added and mixed well with the capture beads, and then incubated at 37°C for 45 min;

Table 2

| Components | Volume added per reaction system (μL) |
|---|---|
| Reverse transcription buffer | 40 |
| dNTPs (10 mM) | 20 |
| Dithiothreitol (DTT, 0.1 M) | 10 |
| Additive (Bead RT/PCR Enhancer) | 12 |
| RNase inhibitor | 10 |
| Reverse transcriptase | 10 |
| Nuclease-free water | 98 |

Exonuclease was added, and incubated at 37°C for 30 min and at 80°C for 20 min, to remove probes that were not attached to mRNA on the surface of capture beads;

Random primer reaction mixture (Table 3) was added, and incubated at 95°C for 5 min, at 1200 rpm at 37°C for 5 min, and at 1200 rpm at 25°C for 15 min; primer extension reaction mixture (Table 4) was added, incubated at 1200 rpm at 25°C for 10 min, at 1200 rpm at 37°C for 15 min, at 1200 rpm at 45°C for 10 min, and at 1200 rpm at 55°C for 10 min, and the amplified single-stranded DNA was eluted with elution buffer;

Table 3

| Components | Volume added per reaction system (μL) |
|---|---|
| Extension Buffer (WTA Extension Buffer) | 20 |
| Random Primers (WTA Extension Primers) | 20 |
| Nuclease-free water | 134 |

Table 4

| Components | Volume added per reaction system (μL) |
|---|---|
| dNTPs (10 mM) | 8 |
| Additive (Bead RT/PCR Enhancer) | 12 |
| Extension Enzyme (WTA Extension Enzyme) | 6 |

Random primers containing universal primers and specific primers (Table 5) were added to the PCR amplification mixture of the extension product. The PCR amplification reaction was performed according to the procedure in Table 6. The amplification product was enriched with random primers, and purified.

Table 5

| Components | Volume added per reaction system (μL) |
|---|---|
| PCR Buffer | 60 |
| universal primer (Universal Oligo) | 10 |
| Specific primer (WTA Amplification Primer) | 10 |

Table 6

| Step | Number of cycles | Temperature(°C) | Time |
|---|---|---|---|
| Hot Start | 1 | 95 | 3 min |
| Denaturation | | 95 | 30 s |
| Annealing | 13 | 60 | 1 min |
| Extension | | 72 | 1 min |

(continued)

| Step | Number of cycles | Temperature(°C) | Time |
|---|---|---|---|
| Final extension | 1 | 72 | 2 min |
| Hold | 1 | 4 | ∞ |

The whole transcriptome index PCR amplification mixture (Table 7) was added, and the PCR amplification reaction was performed according to the reaction procedure in Table 8 (9 cycles of amplification when the molar concentration of the amplification product with random primers was 1-2 nM and 8 cycles of amplification when the molar concentration of the amplification product with random primers was >2 nM). The amplification product was enriched and purified to obtain a single-cell library.

Table 7

| Components | Volume added per reaction system (μL) |
|---|---|
| PCR Buffer | 25 |
| Library Forward Primer | 5 |
| Library Reverse Primer | 5 |
| Nuclease-free water | 5 |

Table 8

| Step | Number of cycles | Temperature(°C) | Time |
|---|---|---|---|
| Hot Start | 1 | 95 | 3 min |
| Denaturation | | 95 | 30 s |
| Annealing | 8/9 | 60 | 30 s |
| Extension | | 72 | 30 s |
| Final extension | 1 | 72 | 1 min |
| Hold | 1 | 4 | ∞ |

IV. Quality test of single-cell library

The concentration of the single-cell library was detected by the Qubit, and the fragment length of the single-cell library was detected by the Agilent 2100 bioanalyzer. It was found that the library concentration was 0.1-100 ng/μL, and the length of the library fragment was about 460-550 bp.

V. Single-cell sequencing

The molar concentration of the single-cell library was calculated to be approximately 1-100 nM according to the concentration and fragment length of the single-cell library. After being diluted to a standard molar concentration of 0.2-2 nM, it was mixed with a balance base library with the same molar concentration at a ratio of a single-cell library: balance base library = 1: (0.05-0.5) for sequencing.

VI. Quality assessment of sequencing data

The amount of total data, Q30, the amount of clustered data, and the amount of effective clustered data of sequencing data were analyzed by BD cwl-runner 3.1. The quality of the sequencing data was simply evaluated.
3057 S1, 2382 S2, and 2683 S3 prepared in Example 1 were sequenced with an average sequencing depth of 50K/cell.

**Example 4 Data Processing**

The following steps 1 and 2 were performed according to the BD Protocol. The BCL file of the sequencing data was converted to a FASTQ-format file, and the quality of the sequencing data was checked and further analysis:

I. Data Preprocessing

Quality control: sequences with a length of read1<60, a length of read2 <42, a base quality of read1 and read2 <20, and SNF of read1 $\geq$ 0.55 or SNF of read2 $\geq$ 0.80 were filtered and removed;
Alignment and annotation: the valid data after quality control was aligned with the reference genome GRCh38, and aligned results were annotated;
RSEC algorithm adjustment: the original matrix file of single-cell gene expression was obtained by continuously performing the algorithm of recursive substitution error correction (RSEC), and correcting the UMI (Unique Molecular Index) information of the alignment results.

II. Noise Correction

The row without a UMI value in the original matrix file of single-cell gene expression was removed. The matrix was transposed to obtain a transposed matrix with columns for different cells and rows for different genes. Each value in the transposed matrix represents the expression level of a certain gene in a certain cell.
Technical noise in the transposed matrix was removed by using the R package "SAVER" (see reference: Huang et al., SAVER: gene expression recovery for single-cell RNA sequencing. Nature Methods (2018) Vol. 15, 539-542), so as to obtain the actual matrix of single-cell gene expression.

III. Standardization

The resulting matrix of single-cell gene expression in step 2 was transposed;
The gene expression level in the matrix of single-cell gene expression was converted into a relative level of transcript expression by using the function "Census" in R package "Monocle 2", thereby accurately obtaining the gene expression levels of all cells, including rare cells and silent cells and forming a standardized matrix of single-cell gene expression in the absence of control sequencing samples.

IV. The acquisition of core transcriptional representation in cell populations

For the standardized matrix of single-cell gene expression obtained in step 3, dimensionality reduction was performed on the first 2000 highly variable genes in the standardized matrix of single-cell gene expression with the method of uniform manifold approximation and projection (UMAP), to obtain 50 non-linearly related and non-biologically function-related projections of the highly variable genes in the low-dimensional space as core transcriptional representations for accurately dividing different cell subpopulations, thus forming a matrix of cell-core transcriptional representation values.

**Example 5 Analysis of the heterogeneity of cell populations**

Based on the matrix of cell-core transcriptional representation values, the score of cell heterogeneity degree in cell populations is calculated according to the following formula:

$$\text{the degree of cell heterogeneity} = \frac{200}{\pi}\tan^{-1}\left(\frac{1}{m}\sum_{i=1}^{m}\sqrt{\sum_{j=1}^{n}(X_{ij} - \mu_j)^2 + 1}\right)$$

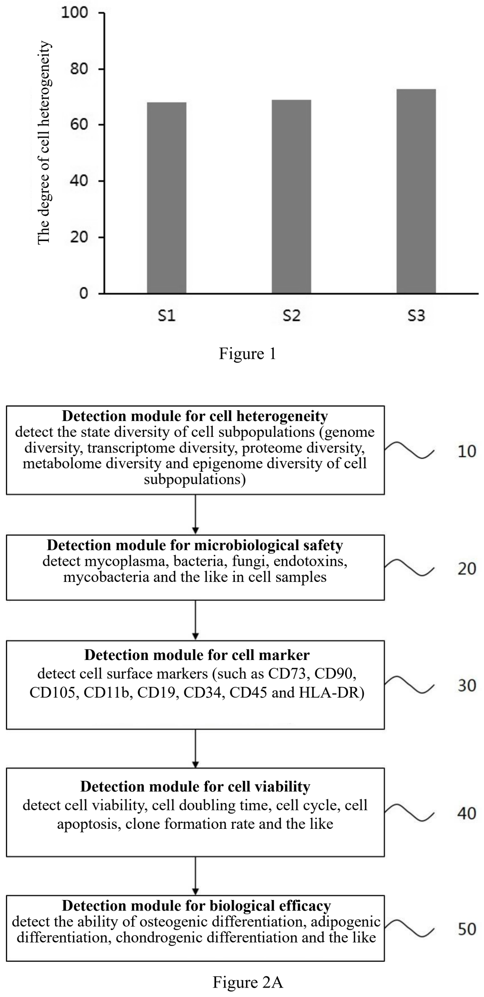

wherein $X_{ij}$ represents the single-cell expression value of the j-th core transcriptional representation in the i-th cell, n represents the number of core transcriptional representations, m represents the cell number in cell populations, and $\mu_j$ represents the mean expression value of the j-th core transcriptional representation in cell populations.
If the single-cell expression values of cells in more than half of the core transcriptional representations exceeded $[\mu_j - 3\sigma_j, \mu_j + 3\sigma_j]$, they were judged as abnormal cells and removed during analysis; wherein $\sigma_j$ represents the standard deviation of the expression value of the j-th core transcriptional representation in cell populations. The value range for the degree of cell heterogeneity is [0,100).
The results were shown in Figure 1. The heterogeneity degrees of S1, S2, and S3 from different donors were different, with heterogeneity degree scores of 67.91, 68.83, and 72.85, respectively.

**Example 6 Safety and efficacy evaluation of cells**

The safety and efficacy of cell samples with different degrees of cell heterogeneity were evaluated, and the feasibility of using cell heterogeneity as a cell quality attribute to evaluate the quality of cell products was explored in the present Example.

I. Safety evaluation

6 8-week-old NCG mice (purchased from Gempharmatech Co., Ltd.) were randomly divided into 4 groups and labeled. Each group consisted of 3 male mice and 3 female mice. The mice were fixed with a fixator. After the injection site of the mice was disinfected, the S1, S2, or S3 suspension was slowly infused into the tail vein of the mice with an infusion dose of $1 \times 10^6$ cells/mouse. A group infused with normal saline was set up as a negative control at the same time.

After cell infusion was completed, the mice were immediately placed in a clean cage and observed by video, and the survival rate of the mice was recorded within 3 minutes.

The results were shown in Table 9. All 6 mice that were infused with S 1 and S2 and 6 mice that were infused with normal saline survived within 3 minutes. 1 mouse that was infused with S3 died within 3 minutes, and 5 mice survived. The heterogeneity degree of S3 is higher than that of S1 and S2, and the safety risk of S3 is also higher than that of S 1 and S2.

Table 9

| Group | Sample | Mice survival/death number | Mice survival rate |
|---|---|---|---|
| Experimental Group | S1 | 6/0 | 100% |
| | S2 | 6/0 | 100% |
| | S3 | 5/1 | 83.3% |
| Negative control group | Normal saline | 6/0 | 100% |

II. Efficacy Evaluation

1. Grouping and drug administration

The SD rat models of permanent ischemic stroke were divided into three groups: the control group of model cells was given normal saline + cyclosporine A (a concentration of 2.5 mg/mL), Group 1 was given cell sample S1 + cyclosporine A, and Group 2 was given cell sample S3 + cyclosporine A, with 8 rats in each group.

After 24 hours of modeling and neurological function scoring, the drug was immediately administered via tail vein injection at a dose of $2 \times 10^7$/kg and a volume of 4 mL/kg. Cyclosporine A was injected intraperitoneally every day before and after modeling. The endpoint of the trial was 28 days after administrating cell therapy.

2. Neurological function scoring

Zea Longa 5-level behavioral scoring and forelimb placement test were performed before medication and 3, 7, 14, 21, and 28 days after medication.

Zea Longa 5-level scoring principle: 0 point, indicates no signs of neurological deficit symptoms; 1 point, indicates mild focal neurological deficit, unable to fully extend the left forelimb; 2 points, indicate moderate focal neurological deficit (rotate to the left); 3 points, indicate severe neurological deficit (leaning to the left); 4 points, indicate no spontaneously walking with reduced level of consciousness.

Measurement of forelimb placing: the examiner holds the skin on the back of the rat so that the limbs are off the ground. The whisker was brushed and touched with the edge of the tabletop corner to test the activity of the forelimb on the same side. For rats whose brains were uninjured, the forelimb can be quickly put on the tabletop, and for rats whose brains were injured, this behavior may cause varying degrees of damage. Each side of the rat was tested 10 times, and the percentage of the times the forelimb touched the edge of the tabletop corner was the score for that side.

The results showed that rats in the control group of the permanent ischemic stroke model showed obvious neurological dysfunction, such as forelimb flexion and rotation towards the hemiplegic side, which recovered slightly over time. Compared with the control group of models, Group 1 showed a significant decrease in neurological function scores ($P<0.05$-$0.01$) after 14-28 days of medication ($P<0.05$-$0.01$), with an improvement rate of 38.6% after 28 days of medication ($P<0.05$); Group 2 showed a significant decrease in neurological function scores (P<0.05) after 14-28 days of medication (P<0.05-0.01), with an improvement rate of 30.7% after 28 days of medication ($P<0.05$) (compared with Group 1 and Group 2: $P>0.05$).

It was found that the treatment effect of Group 1 was slightly better than that of Group 2 (the improvement rates after 28 days of medication were 38.6% vs 30.7%, $P>0.05$) by the comparison of the improvement rate of neurological function scores after 14-28 days of medication. The results were shown in Table 10 (*$P<0.05$, **$P<0.01$).

Table 10 Effects of cells on neurological function scores in rats with permanent ischemic stroke ($\bar{x} \pm$s, n=8)

| Group | Dose | Before medication (score) | 3 days after medication (score) | 7 days after medication (score) | 14 days after medication (score) | 21 days after medication (score) | 28 days after medication (score) | Improvement rate after 28 days of medication (%) |
|---|---|---|---|---|---|---|---|---|
| Model control group | - | 2±0 | 2.00±0 | 1.63±0.52 | 1.75±0.46 | 1.75±0.46 | 1.63±0.52 | - |
| Group 1 | 2×10$^7$/kg | 2±0 | 1.88±0.35 | 1.50±0.53 | 1.25±0.46* | 1.13±0.35** | 1.00±0.53* | 38.6% |
| Group 2 | 2×10$^7$/kg | 2±0 | 2.00±0 | 1.38±0.52 | 1.25±0.46* | 1.25±0.46* | 1.13±0.64(P=0.09) | 30.7% |

Rats in the control group of the permanent ischemic stroke model showed brain injury and placement disorder of the contralateral forelimb. Compared with the control group of model, the placement rates of the forelimb in Groups 1 and 2 were significantly improved after 21-28 days of medication ($P<0.01$-$0.001$), with improvement rates of 48.7% ($P<0.001$) and 44.1% ($P<0.001$) respectively after 28 days of medication.

It was found that the treatment effect of Group 1 was slightly better than that of Group 2 (the improvement rates after 28 days of medication were 48.7% vs 44.1%, *P>0.05)* by comparison of placement rates of the forelimb after 21-28 days of medication. The results were shown in Table 11 *(\*P<0.05, \*\*P<0.01, \*\*\*P<0.001).*

Table 11 Effects of cells on the contralateral forelimb placement (%) in rats with permanent ischemic stroke ($\bar{x} \pm s$, n=8)

| Group | Dose | Before medication (score) | 3 days after medication (score) | 7 days after medication (score) | 14 days after medication (score) | 21 days after medication (score) | 28 days after medication (score) | Improvement rate after 28 days of medication (%) |
|---|---|---|---|---|---|---|---|---|
| Model control group | - | 3.8±5.2 | 8.8±3.5 | 12.5±8.9 | 33.8±10.6 | 45.0±10.7 | 53.8±11.9 | - |
| Group 1 | $2\times10^7$/kg | 3.8±5.2 | 8.8±8.3 | 22.5±14.9 | 46.3±15.1 | 76.3±16.9** | 80.0±10.7*** | 48.7% |
| Group 2 | $2\times10^7$/kg | 3.8±7.4 | 7.5±8.9 | 22.5±12.8 | 46.3±16.9 | 72.5±21.2** | 77.5±13.9*** | 44.1% |

3. Determination of cerebral infarction area

After 28 days of medication, the rats were bled and their brains were collected. The brain tissues were frozen in a -20°C freezer and then sliced with a thickness of 2 mm per slice. The brain tissue slices were placed in a 2% red tetrazolium (TTC) solution and incubated at 37°C for 5 min. The infarcted tissue appeared white, and the non-infarcted tissue appeared red. Image J software was used to measure the area of cerebral infarction, and the percentage of cerebral infarction area to the whole brain area was calculated.

In order to exclude the deviation caused by cerebral edema or brain atrophy, the following formula was used to calculate the area of cerebral infarction (%):

area of cerebral infarction (%) = (area of healthy hemisphere - area of normal brain in infarcted side) / (area of healthy hemisphere $\times$ 2) $\times$ 100%

Rats in the control group of the permanent ischemic stroke model showed obvious cerebral infarction. Compared with the control group of model, the area of cerebral infarction in Groups 1 and 2 was significantly reduced after 28 days of medication (P<0.05), with improvement rates of 19.2% (P<0.05) and 15.7% (P<0.05), respectively.

It was found that the treatment effect of Group 1 was slightly better than that of Group 2 (the improvement rates were 19.2% vs 15.7%, *P>0.05)* by comparison of cerebral infarction area after 28 days of medication.

Table 12 Effects of cells on the area of cerebral infarction in rats with permanent ischemic stroke ( $\bar{x} \pm s$, n=8)

| Group | Dose | Cerebral infarction (%) | Improvement rate (%) |
|---|---|---|---|
| Model control group | - | 28.7$\pm$4.1 | - |
| Group 1 | 2$\times$10$^7$/kg | 23.2$\pm$4.0* | 19.2 |
| Group 2 | 2$\times$10$^7$/kg | 24.2$\pm$3.7* | 15.7 |

Combined with the above evaluation results, the degree of heterogeneity in cell populations is correlated with the safety and efficacy of cells. Cell heterogeneity, as a cell quality attribute, can be used to evaluate cell quality.

**Example 7 Device for detecting cell quality attributes**

As shown in Figure 2A, the present example provided a detection device for cell quality attributes. the detection device included a detection module for cell heterogeneity 10, a detection module for microbiological safety 20, a detection module for cell marker 30, a detection module for cell viability 40, and a detection module for biological efficacy 50;

the state diversity of cell subpopulations (genome diversity, transcriptome diversity, proteome diversity, metabolome diversity, and epigenome diversity of cell subpopulations) was detected by the detection module for cell heterogeneity 10;

mycoplasma, bacteria, fungi, endotoxins, mycobacteria, and the like in cell samples were detected by the detection module for microbiological safety 20;

cell surface markers (such as CD73, CD90, CD105, CD11b, CD19, CD34, CD45, and HLA-DR) were detected by the detection module for cell marker 30;

cell viability, cell doubling time, cell cycle, cell apoptosis, clone formation rate and the like were detected by the detection module for cell viability 40; and

the ability of osteogenic differentiation, adipogenic differentiation, chondrogenic differentiation and the like were detected by the detection module for biological efficacy 50.

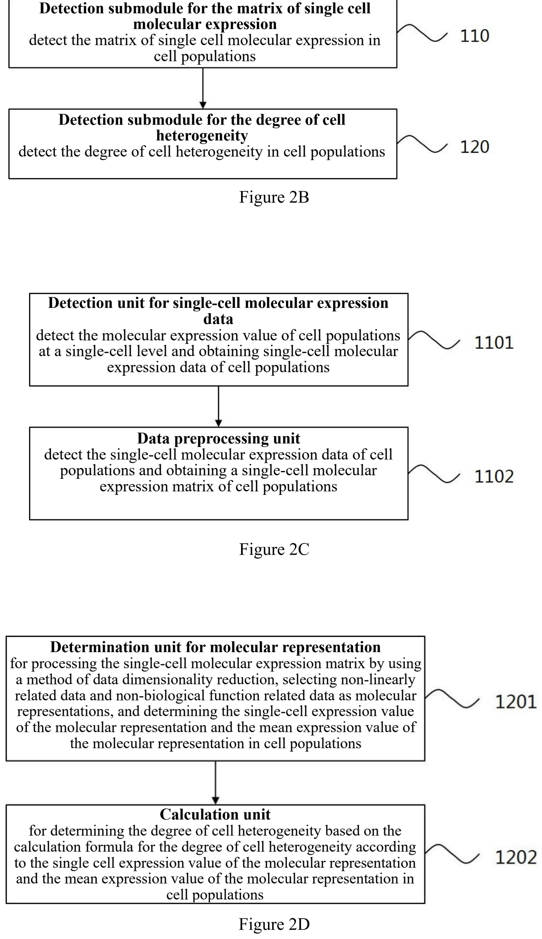

As shown in Figure 2B, the detection module for cell heterogeneity 10 includes:

a detection submodule for the matrix of single-cell molecular expression 110 for detecting the matrix of single-cell molecular expression in cell populations; and

a detection submodule for the degree of cell heterogeneity 120 for detecting the degree of cell heterogeneity in cell populations.

As shown in Figure 2C, the detection submodule for the matrix of single-cell molecular expression 110 includes:

a detection unit for single-cell molecular expression data 1101 for detecting the molecular expression value of cell populations at a single-cell level and obtaining single-cell molecular expression data of cell populations; and

a data preprocessing unit 1102 for detecting the single-cell molecular expression data of cell populations and obtaining a single-cell molecular expression matrix of cell populations; wherein the preprocessing includes noise correction and/or standardization.

As shown in Figure 2D, the detection submodule for the degree of cell heterogeneity 10 includes:

a determination unit for molecular representation 1201 for processing the single-cell molecular expression matrix by using a method of data dimensionality reduction, selecting non-linearly related data and non-biological function-related data as molecular representations, and determining the single-cell expression value of the molecular representation and the mean expression value of the molecular representation in cell populations; wherein the molecular representation is selected from the gene representation, transcriptional representation, protein representation, metabolite representation or epigenetic site representation that can represent single-cell molecular expression matrix of cell populations;

a calculation unit 1202 for determining the degree of cell heterogeneity based on the calculation formula for the degree of cell heterogeneity according to the single cell expression value of the molecular representation and the mean expression value of the molecular representation in cell populations;

the calculation formula for the degree of cell heterogeneity is as follows:

$$\text{the degree of cell heterogeneity} = \frac{200}{\pi}\tan^{-1}\left(\frac{1}{m}\sum_{i=1}^{m}\sqrt{\sum_{j=1}^{n}(X_{ij}-\mu_j)^2 + 1}\right)$$

wherein $X_{ij}$ represents the single-cell expression value of the j-th molecular representation in the i-th cell, n represents the number of molecular representations, m represents the cell number in cell populations, and $\mu_j$ represents the mean expression value of the j-th molecular representation in cell populations.

**Example 8 Development of preparation process for ADSCs seed bank**

In the present Example, process development was performed on the washing times of primary ADSCs during the production of the ADSCs seed bank, and the production process parameters were confirmed by calculating the degree of cell heterogeneity of the ADSCs seed bank samples.

12 50-mL Falcon tubes were prepared in advance and labeled into four groups with three tubes in each group for later use;

a sample of adipose tissue collected within 24 hours was transferred from the collection bottle to a 50-mL Falcon tube, twice the volume of normal saline was added, then shaken vigorously for 20 seconds and let stand for 5 minutes. After the adipose tissue and normal saline were completely layered, the liquid layer below the tissue was aspirated and discarded. The adipose tissue was repeatedly rinsed with normal saline until no obvious red color in the liquid layer below the tissue;

20 mL of each aliquot of the washed adipose tissue was added to a 50-mL Falcon tube, and an equal volume of dPBS was added, and then centrifuged at 400 g for 5 min. The solution was divided into an upper layer of lipid, a middle layer of lipid tissue, a lower layer of dPBS, and a blood cell pellet. The upper layer of lipid, bottom layer of dPBS, and blood cell pellet were removed;

twice the volume of 1 mg/mL type I collagenase (tissue digestion solution) was added to the adipose tissue, transferred to a thermostatic air shaker preheated at 37°C, and digested at 120 rpm/min for 1 h;

the digested tissue was centrifuged at 500 g for 8 min at room temperature. The solution was divided into an upper layer of lipid, a middle layer of lipid tissue, a lower layer of digestion solution, and a bottom layer of cell pellet after centrifugation. The upper layer of lipid, middle layer of lipid tissue, and lower layer of digestion solution were aspirated and discarded. The cell pellet was resuspended with dPBS, filtered into a 50-mL Falcon tube with a 100-μm sieve, and centrifuged at 500 g for 5 min. The supernatant was removed to obtain a cell pellet containing P0 ADSCs;

the cell pellet was resuspended with dPBS and was added into 12 Falcon tubes after mixing by pipetting up and down. The pellet was washed with dPBS once, twice, three times, or four times by groups, and centrifuged at 500 g for 5 min. The supernatant was removed; and

after the primary culture of P0 ADSCs using a complete medium, they were passaged to P2, and the degree of cell heterogeneity of P0, P1, and P2 cells was detected under different washing times of primary ADSCs.

The above experiments were conducted using adipose tissue from three donors.

The results of the degree of cell heterogeneity test were shown in Table 13. The primary ADSCs of the same batch of samples were washed once before inoculation, and many miscellaneous cells remained. As the subculture progressed, the degree of cell heterogeneity of P0-P2 samples changed significantly, and the consistency of heterogeneity was not maintained, making it impossible to obtain an ADSCs seed bank with stable quality. The primary ADSCs were washed 2, 3, or 4 times before inoculation, and the degree of cell heterogeneity of P0-P2 samples remained consistent without significant statistical differences, resulting in an ADSCs seed bank with stable quality. There was consistency in the degree of cell heterogeneity in the three batches of P0-P2 samples.

Considering the effect of washing times on cell viability, during the production of the ADSCs seed bank, primary ADSCs were washed twice with dPBS, and then inoculated and passaged to prepare the ADSCs seed bank.

Table 13

| Batch | Washing times | Degree of cell heterogeneity (mean ± sd) | | |
|---|---|---|---|---|
| | | P0 | P1 | P2 |
| First batch | 1 | 65.65 ±3.13 | 71.63±4.32 | 78.36±5.83 |
| | 2 | 68.65 ±4.63 | 70.33±4.72 | 73.18±5.02 |
| | 3 | 70.35 ±3.91 | 71.63±4.32 | 70.36±5.83 |
| | 4 | 69.74±4.21 | 68.61±3.87 | 71.87±4.86 |
| Second batch | 1 | 63.77 ±4.32 | 74.02±4.19 | 81.12±4.90 |
| | 2 | 69.89 ±3.03 | 71.12±5.61 | 70.08±2.89 |
| | 3 | 67.91 ±4.32 | 71.16±3.65 | 71.67±5.23 |
| | 4 | 67.37±3.89 | 71.51±5.12 | 71.27±3.86 |
| Third batch | 1 | 64.85 ±4.12 | 72.61±4.65 | 79.40±4.71 |
| | 2 | 66.26 ±2.94 | 69.43±4.56 | 67.18±3.75 |
| | 3 | 66.93 ±3.47 | 66.63±3.86 | 66.13±5.49 |
| | 4 | 69.83±3.721 | 69.81±4.23 | 70.73±4.09 |

**Example 9 Stability study of ADSCs seed bank**

In the present Example, the degree of cell heterogeneity of ADSCs seed bank samples was calculated to explore the temporal variation of quality attributes of ADSCs seed bank under liquid nitrogen storage conditions, providing a scientific basis for the production, packaging, storage, transportation conditions, and retesting period of products.

9 50-mL Falcon tubes were prepared in advance, and labeled into three groups with three tubes in each group. 10 mL of normal saline containing 1% human albumin was added to each tube for later use;

a total of 9 tubes of ADSCs seed bank samples were randomly selected from three batches in the liquid nitrogen tank, with 3 tubes in each batch, and placed in a thermostat water bath at 37°C. They were gently shaken to thaw cells evenly and then were transferred to a biosafety cabinet. All cells from the same batch were aspirated and pipetted into a 50-mL Falcon tube. After being mixed by pipetting up and down, they were evenly added to a Falcon tube pre-added with normal saline containing 1% human albumin, and washed.

the Falcon tube was placed in a centrifuge and centrifuged at 400 g for 5 min at room temperature; after centrifugation, the Falcon tube was taken from the centrifuge and transferred to a biosafety cabinet. The supernatant was aspirated and discarded, and 10 mL of normal saline containing 1% human albumin was added to each tube for resuspending, pipetting, and washing cells;

the Falcon tube was placed in a centrifuge and centrifuged at 400 g for 5 min at room temperature; after centrifugation, the Falcon tube was taken from the centrifuge and transferred to a biosafety cabinet. The supernatant was aspirated and discarded, and 10 mL of normal saline containing 1% human albumin was added to each tube for mixing and pipetting cells; They were filtered with a 100-μm cell sieve into a new 50-mL Falcon tube, samples were taken and counted separately. Normal saline containing 1% human albumin was added to adjust cell concentration to the same level according to the counting results; and

the degree of cell heterogeneity was detected at 0th, 3rd, 6th, 9th and 12th month after the preparation of the ADSCs seed bank.

The results of the degree of cell heterogeneity test were shown in Table 14. There was no significant statistical difference between the degree of cell heterogeneity of samples in the same batch tested in the 3rd, 6th, 9th and 12th months and the degree of cell heterogeneity of that tested in the 0th month. The consistency of the degree of cell heterogeneity test data among three batches of samples was food.

It indicated that the ADSCs seed bank stored in liquid nitrogen maintained stable quality within one year.

Table 14

| Batch | Degree of cell heterogeneity (mean $\pm$ sd) | | | | |
| --- | --- | --- | --- | --- | --- |
| | 0th month | 3rd month | 6th month | 9th month | 12th month |
| First batch | 73.34$\pm$1.85 | 73.57$\pm$1.76 | 74.31$\pm$1.69 | 74.40$\pm$1.65 | 74.52$\pm$1.41 |
| Second batch | 73.14$\pm$1.63 | 73.21$\pm$1.64 | 73.92$\pm$1.78 | 74.37$\pm$1.74 | 74.42$\pm$1.51 |
| Third batch | 73.23$\pm$1.45 | 73.38$\pm$1.70 | 74.09$\pm$1.55 | 74.29$\pm$1.52 | 74.34$\pm$1.42 |

**Example 10 Development of preparation process for ADSCs working bank**

In the present Example, process development was conducted for the time of cell contact with the cryoprotectant during the production of the ADSCs working bank, and the production process parameters were confirmed by calculating the degree of cell heterogeneity of ADSCs working bank samples.

12 cryopreservation tubes were prepared in advance, and labeled into four groups with three tubes in each group. Cryoprotectant was added to each tube for later use;

1 tube of ADSCs seed bank sample was randomly selected from the liquid nitrogen tank, and placed in a thermostat water bath at 37°C. It was gently shaken to thaw cells evenly and then was transferred to a biosafety cabinet. All cells were aspirated and pipetted into a Falcon tube pre-added with normal saline containing 1% human albumin and were washed 3 times.

the Falcon tube was placed in a centrifuge and centrifuged at 400 g for 5 min at room temperature; after centrifugation, the Falcon tube was taken from the centrifuge and transferred to a biosafety cabinet. The supernatant was aspirated and discarded, and 10 mL of normal saline containing 1% human albumin was added to each tube for mixing and pipetting cells, and for cell counting;

cells were evenly inoculated into 4 T75 flasks for cell culture at a density of $5\times 10^3$ cells/cm$^2$, and a complete medium was added to 10 mL/flask. Cells were transferred to an incubator for cell culture, and incubated at 37°C and 5% CO$_2$; when cells were 90% confluence, subculture was performed and repeated until P5;

when P5 cells were 80% confluence, a T75 flask of cells was digested, the cell pellet was collected. Cells were added into three cryopreservation tubes containing cryoprotectant at a mean density of $1\times 10^7$ cells/mL/tube, and then the timer was started;

in the sequence of 3h, 2h, 1h, and 0h of contact time between cells and cryoprotectant, the cells in the remaining three T75 flasks were digested and collected, and added evenly into three cryopreservation tubes containing freezing solution; and

the operation of each step was integrated to ensure each group reached the time point simultaneously and performed programmed cooling and cryopreservation; the cells were recovered after 7 days of cryopreservation, and the degree of cell heterogeneity of each group of cells was detected before the addition of cryoprotectant before programmed cooling and cryopreservation, and recovery after 7 days of cryopreservation.

The above trials were completed by using three batches of ADSCs seed banks.

It was found that when the same batch of samples was in contact with the cryoprotectant for more than 2 hours before cryopreservation, it significantly affected the transcriptome state of cells, resulting in significant changes in the degree of cell heterogeneity before and after cryopreservation, making it impossible to obtain an ADSCs working bank with stable quality. There was consistency in the changes in the degree of cell heterogeneity among the three batches of samples before and after cryopreservation.

Therefore, in the production process of ADSCs working bank, it is necessary to limit the P5 cells to enter the procedure of programmed cooling and cryopreservation within 2 hours after contacting the cryoprotectant.

**Example 11 Stability study of ADSCs working bank**

In the present Example, the degree of cell heterogeneity of ADSCs working bank samples was calculated to explore the temporal variation of quality attributes of ADSCs working bank under liquid nitrogen storage conditions, providing a scientific basis for the production, packaging, storage, transportation conditions, and retesting period of products.

I. Storage stability

9 50-mL Falcon tubes were prepared in advance, and labeled into three groups with three tubes in each group. 10 mL of normal saline containing 1% human albumin was added to each tube for later use;

a total of 9 tubes of ADSCs working bank samples were randomly selected from three batches in the liquid nitrogen tank, with 3 tubes in each batch, and placed in a thermostat water bath at 37°C. They were gently shaken to thaw cells evenly and then were transferred to a biosafety cabinet. All cells from the same batch were aspirated and pipetted into a 50-mL Falcon tube. After being mixed by pipetting up and down, they were evenly added to a Falcon tube pre-added with normal saline containing 1% human albumin, and washed.

the Falcon tube was placed in a centrifuge and centrifuged at 400 g for 5 min at room temperature; after centrifugation, the Falcon tube was taken from the centrifuge and transferred to a biosafety cabinet. The supernatant was aspirated and discarded, and 10 mL of normal saline containing 1% human albumin was added to each tube for resuspending, pipetting, and washing cells;

the Falcon tube was placed in a centrifuge and centrifuged at 400 g for 5 min at room temperature; after centrifugation, the Falcon tube was taken from the centrifuge and transferred to a biosafety cabinet. The supernatant was aspirated and discarded, and 10 mL of normal saline containing 1% human albumin was added to each tube for mixing and pipetting cells; They were filtered with a 100-μm cell sieve into a new 50-mL Falcon tube, samples were taken and counted separately. Normal saline containing 1% human albumin was added to adjust cell concentration to the same level according to the counting results;

the degree of cell heterogeneity was detected at 0th, 3rd, 6th, 9th and 12th month after the preparation of the ADSCs working bank.

The results showed that there was no significant statistical difference between the degree of cell heterogeneity of samples in the same batch tested in the 3rd, 6th, 9th and 12th months and the degree of cell heterogeneity of that tested in the 0th month. The consistency of the degree of cell heterogeneity test data among three batches of samples was food.

It indicated that the ADSCs working bank stored in liquid nitrogen maintained stable quality within one year.

II. Passage stability

The samples for the passage stability test were two batches of ADSCs working bank randomly selected from the liquid nitrogen tank, and the cryopreservation samples of cells that were subcultured to P7-P10 after their recovery using the same production process.

A total of 6 tubes of ADSCs working bank samples were randomly selected from two batches in the liquid nitrogen tank, with 3 tubes in each batch. They were passaged to P10 after their recovery using the same production process. During the passage process, P7 and P10 cells were retained for cryopreservation and stored in liquid nitrogen;

After the preparation of P5, P7, and P10 cell cryopreservation samples, they were immediately recovered and the degree of cell heterogeneity was detected.

As shown in Figure 3, it was the score distribution of cell heterogeneity degree for P5, P7, and P10 ADSCs samples in two batches (A1 and A2); as shown in Table 15, Figure 4A, Figure 4B and Figure 4C, it can be seen that the degree of cell heterogeneity followed a normal distribution among ADSCs samples of different passages and batches.

Table 15

| Batch | Passage | Variable | Probability | P-value |
|---|---|---|---|---|
| A1 | P5 | The degree of cell heterogeneity | 0.9930 | 0.9722 |
| A1 | P7 | The degree of cell heterogeneity | 0.9153 | 0.5109 |
| A1 | P10 | The degree of cell heterogeneity | 0.9528 | 0.7339 |
| A2 | P5 | The degree of cell heterogeneity | 0.8770 | 0.3262 |

(continued)

| Batch | Passage | Variable | Probability | P-value |
|---|---|---|---|---|
| A2 | P7 | The degree of cell heterogeneity | 0.7576 | 0.0453 |
| A2 | P10 | The degree of cell heterogeneity | 0.7955 | 0.0944 |

A variance analysis was conducted on the degree of cell heterogeneity for P5, P7, and P10 ADSCs samples from two batches (A1 and A2). The results were shown in Figure 5A and Figure 5B. There was no significant statistical difference in the degree of cell heterogeneity between ADSCs samples of the same batch but different passages; there was also no significant statistical difference in the degree of cell heterogeneity between ADSCs samples of the same passage but different batches.

It indicated that the ADSCs working bank stored in liquid nitrogen maintained stable quality between passages.

The 95% and 99% confidence intervals of the scores of cell heterogeneity degree at different passages were used as the reference ranges for the degree of cell heterogeneity of ADSCs with stable quality by using the t-test method. The specific results were shown in Figure 6A, Figure 6B, Figure 6C, and Figure 6D.

**Example 12 Development of production process for ADSCs injection**

In the present Example, process development was conducted on the excipient proportion during the production of ADSCs injection, and the production process parameters were confirmed by calculating the degree of cell heterogeneity of ADSCs injection samples.

45 50-mL Falcon tubes were prepared in advance and 30 mL of normal saline containing 1% human albumin was added to each tube for later use;

135 tubes of ADSCs working bank samples in the same batch were randomly selected from the liquid nitrogen tank, and placed in a thermostat water bath at 37°C. They were gently shaken to thaw cells evenly and then were transferred to a biosafety cabinet. All cells were aspirated and pipetted into a 50-mL Falcon tube. After being mixed by pipetting up and down, they were evenly added to 45 Falcon tubes pre-added with normal saline containing 1% human albumin, and washed.

the Falcon tubes were placed in a centrifuge and centrifuged at 400 g for 5 min at room temperature; after centrifugation, the Falcon tube was taken from the centrifuge and transferred to a biosafety cabinet. The supernatant was aspirated and discarded, and 30 mL of normal saline containing 1% human albumin was added to each tube for mixing and pipetting cells, and for cell counting;

the Falcon tubes were placed in a centrifuge and centrifuged at 400 g for 5 min at room temperature; after centrifugation, the Falcon tube was taken from the centrifuge and transferred to a biosafety cabinet. The supernatant was aspirated and discarded, and all Falcon tubes were divided into 9 groups according to the labels in Table 16, with 5 tubes in each group.

Table 16

| Group | $1\times10^6$/mL | $2\times10^6$/mL | $5\times10^6$/mL |
|---|---|---|---|
| normal saline containing 0.5% human albumin | 1 | 2 | 3 |
| normal saline containing 1% human albumin | 4 | 5 | 6 |
| normal saline containing 2% human albumin | 7 | 8 | 9 |

According to Table 16, 30 mL of normal saline containing corresponding concentrations of human albumin was added to samples in each group. It was mixed by pipetting up and down, filtered with a 100-$\mu$m cell sieve into a new 50 mL Falcon tube, and samples were taken and counted separately. Normal saline containing human albumin was added to adjust cell concentration to the concentration required by the grouping according to the counting results;

The cells were infused into 100-mL disposable blood bags, with $5\times10^7$ cells in each bag and three bags per group, to prepare preparations for human ADSCs injection, which were stored at 4°C;

The degree of cell heterogeneity was detected at 0 h, 12 h, and 24 h after the preparation was prepared, and one bag of preparation was taken from each group at a time.

The above trials were conducted by selecting three batches of ADSCs working bank samples with similar degrees of cell heterogeneity.

The results of the cell heterogeneity test are shown in Table 17. When using normal saline containing 1% human albumin as an excipient and a cell concentration of $1\times10^6$ cells/mL, the cell heterogeneity of samples in the same batch was consistent within 24 hours, and the cell heterogeneity of samples in different batches was consistent. Therefore, in the production process of human ADSCs injection, 5% human albumin (20%): 95% normal saline was selected as the most suitable excipient ratio, and $1 \times 10^6$ cells/mL was selected as the most suitable cell concentration.

Table 17

| Group | | The degree of cell heterogeneity | | |
|---|---|---|---|---|
| | | 0 h | 12 h | 24 h |
| First batch | 1 | 77.31 | 74.83 | 75.34 |
| | 2 | 75.8 | 73.32 | 80.56 |
| | 3 | 74.61 | 76.99 | 82.88 |
| | 4 | 76.45 | 76.64 | 72.04 |
| Second batch | 1 | 64.72 | 72.36 | 75.66 |
| | 2 | 77.04 | 77.59 | 75.69 |
| | 3 | 77.44 | 79.79 | 76.64 |
| | 4 | 71.54 | 35.68 | 79.52 |
| Third batch | 1 | 73.23 | 81.76 | 75.80 |
| | 2 | 76.42 | 77.06 | 82.20 |
| | 3 | 83.94 | 74.63 | 77.31 |
| | 4 | 72.84 | 74.98 | 73.21 |

**Example 13 Study on the stability of ADSCs injection**

In the present Example, the degree of cell heterogeneity of ADSCs injection samples was calculated to explore the temporal variation of quality attributes of ADSCs injection during storage, transportation, and use, providing a scientific basis for determining the prescription, storage, and transportation conditions, and the validity term of preparations.

I. Storage stability

A total of 18 bags of ADSCs injection from three batches were randomly selected, with 6 bags in each batch, and let them stand at $5\pm3°C$ for 24 hours;
At the 6th and 24th hour, 3 bags of preparations from each batch were taken to detect the degree of cell heterogeneity.
As shown in Figure 7A, it was the score distribution of cell heterogeneity degree for ADSCs injection left at $5\pm3°C$ for 6 h or 24 h. As shown in Table 18 and Figure 8A, it can be seen that the degree of cell heterogeneity for ADSCs injection followed a normal distribution when they were left at $5\pm3°C$ for 6 h or 24 h.

Table 18

| Storage time | Transport conditions | Use conditions | Variable | Probability | P-value |
|---|---|---|---|---|---|
| 6h | Low speed (100 rpm/min) | $5\pm3°C$ | The degree of cell heterogeneity | 0.99 | 0.97 |
| 6h | Low speed (100 rpm/min) | Room temperature | The degree of cell heterogeneity | 0.92 | 0.51 |
| 6h | High speed (300 rpm/min) | $5\pm3°C$ | The degree of cell heterogeneity | 0.85 | 0.24 |
| 6h | High speed (300 rpm/min) | Room temperature | The degree of cell heterogeneity | 0.78 | 0.08 |
| 24 h | Low speed (100 rpm/min) | $5\pm3°C$ | The degree of cell heterogeneity | 0.88 | 0.33 |
| 24 h | Low speed (100 rpm/min) | Room temperature | The degree of cell heterogeneity | 0.80 | 0.10 |

(continued)

| Storage time | Transport conditions | Use conditions | Variable | Probability | P-value |
|---|---|---|---|---|---|
| 24 h | High speed (300 rpm/min) | 5±3°C | The degree of cell heterogeneity | 0.95 | 0.74 |
| 24 h | High speed (300 rpm/min) | Room temperature | The degree of cell heterogeneity | 0.73 | 0.02 |

II. Transportation stability

A total of 18 bags of ADSCs injection from three batches were randomly selected, with 6 bags in each batch, and placed at 5±3°C, shaken at low speed (100 rpm/min) or high speed (300 rpm/min) for 12 hours;

At the 12th hour, 3 bags of preparations from each batch were taken to detect the degree of cell heterogeneity.

As shown in Figure 7B, it was the score distribution of cell heterogeneity degree for ADSCs injection agitated at 5±3°C at low speed (100 rpm/min) or high speed (300 rpm/min). As shown in Table 18 and Figure 8B, it can be seen that the degree of cell heterogeneity for ADSCs injection followed a normal distribution when they were agitated at 5±3°C at low speed (100 rpm/min) or high speed (300 rpm/min).

III. Stability in use

A total of 18 bags of ADSCs injection from three batches were randomly selected, with 6 bags in each batch, and let them stand at 5±3°C for 2 hours or at room temperature (10-30°C) for 2 hours;

At the 2nd hour, 3 bags of preparations from each batch were taken to detect the degree of cell heterogeneity.

As shown in Figure 7C, it was the score distribution of cell heterogeneity degree for ADSCs injection left at 5±3°C or room temperature (10-30°C) for 2h. As shown in Table 18 and Figure 8CA, it can be seen that the degree of cell heterogeneity for ADSCs injection followed a normal distribution when they were left at 5±3°C or room temperature (10-30°C) for 2 h.

IV. Joint stability

Based on the above test results of cell heterogeneity, the stability of ADSCs injection under the combined influence of multiple factors was analyzed.

As shown in Figure 9, the degree of cell heterogeneity of ADSCs injection followed a normal distribution under the combined influence of multiple factors. The variance analysis was performed on the degree of cell heterogeneity under the combined influence of multiple factors. The results were shown in Table 19, there was no significant statistical difference in the degree of cell heterogeneity under the influence of multiple factors. The interactive effects among multiple factors were analyzed. As shown in Figure 10, the storage time and use conditions had an interactive effect on the degree of cell heterogeneity. There was no significant statistical difference in the degree of cell heterogeneity affected by the combined effects of any two factors. The results were shown in FIGS. 11A, 11B, 11C, 11D, 11E, and 11F.

It indicated that the ADSCs injection maintained stable quality under the combined influence of storage time, transportation conditions, and use conditions.

Table 19

| | Df | Sum Sq | Mean Sq | F-value | Pr(>F) |
|---|---|---|---|---|---|
| Storage time | 1.000 | 240.792 | 240.792 | 3.398 | 0.078 |
| Transportation conditions | 1.000 | 56.818 | 56.818 | 0.802 | 0.379 |
| Use condition | 1.000 | 69.443 | 69.443 | 0.980 | 0.332 |
| Storage time: transportation conditions | 1.000 | 18.120 | 18.120 | 0.256 | 0.618 |
| Storage time: use conditions | 1.000 | 122.383 | 122.383 | 1.727 | 0.201 |
| Transport conditions: use conditions | 1.000 | 23.805 | 23.805 | 0.336 | 0.568 |
| Storage time: transportation conditions: use conditions | 1.000 | 86.461 | 86.461 | 1.220 | 0.280 |
| Residual value | 24.000 | 1700.893 | 70.871 | NA | NA |

The 95% and 99% confidence intervals of the scores of cell heterogeneity degree at different storage time were used as the reference ranges for the degree of cell heterogeneity of ADSCs with stable quality by using the t-test method. The specific results were shown in Figure 12A, Figure 12B, Figure 12C, and Figure 12D.

The applicant declares that the present application is illustrated and explained through the above embodiments, but the present application is not limited to the above embodiments, that is, it does not mean that the present application must rely on the methods, steps, or devices in the above embodiments to be implemented. It is apparent that many modifications and variations can be made by a person skilled in the art in light of the above teachings. It should be understood to a person skilled in the art that any improvements to the present application fall within the protection scope and disclosure scope of the present application. It is intended that the scope of the application and all equivalents are defined by the following claims.

**Industrial Applicability**

The present application provides a method, a device, and a use for detecting cell quality attributes. The detection method includes the step of detecting cell heterogeneity, wherein the cell heterogeneity includes state diversity of cell subpopulations, wherein the state diversity of cell subpopulations includes any one or a combination of at least two of genome diversity, transcriptome diversity, proteome diversity, metabolome diversity and epigenome diversity of cell subpopulations. The present application uses the heterogeneity of cells affected by the microenvironment as a cell quality attribute. Compared with the currently commonly used cell quality assessment indicators, it can more accurately assess the quality of cell products at the single-cell level. The detection method of a cell quality attribute established thereby plays an important role in accurately detecting the quality of cell products and ensuring the stability of clinical treatment effects and has good economic value and application prospects.

**Claims**

1. A detection method of a cell quality attribute, wherein the detection method comprises a step of detecting cell heterogeneity, wherein the cell heterogeneity comprises state diversity of cell subpopulations, wherein the state diversity of cell subpopulations comprises any one or a combination of at least two of genome diversity, transcriptome diversity, proteome diversity, metabolome diversity and epigenome diversity of cell subpopulations.

2. The detection method of claim 1, wherein the detection method comprises a step of detecting the degree of cell heterogeneity, which comprises:

   determining a molecular representation and a single-cell expression value of the molecular representation based on a single-cell molecular expression matrix of cell populations;
   determining a mean expression value of the molecular representation in cell populations; and
   determining the degree of cell heterogeneity based on the single-cell expression value of the molecular representation and the mean expression value of the molecular representation in cell populations.

3. The detection method of claim 2, wherein the method for obtaining the single-cell molecular expression matrix of cell populations comprises:

   detecting a molecular expression value of cell populations at a single-cell level to obtain single-cell molecular expression data of cell populations; and
   preprocessing the single-cell molecular expression data of cell populations to obtain a single-cell molecular expression matrix of cell populations;
   wherein the preprocessing comprises noise correction and/or standardization.

4. The detection method of claim 2, wherein the method for determining the molecular representation comprises:
   processing the single-cell molecular expression matrix by using a method of data dimensionality reduction, and selecting non-linearly related data and non-biological function related data as molecular representations.

5. The detection method of claim 2, wherein the molecular representation is selected from any one or a combination of at least two of gene representation, transcriptional representation, protein representation, metabolite representation, and epigenetic site representation, which can represent single-cell molecular expression of cell populations.

6. The detection method of claim 2, wherein a Euclidean distance is calculated based on the single-cell expression value of the molecular representation and the mean expression value of the molecular representation in cell populations, and the degree of cell heterogeneity is determined.

7. The detection method of claim 2, wherein the degree of cell heterogeneity is determined based on the calculation formula for the degree of cell heterogeneity according to the single-cell expression value of the molecular representation and the mean expression value of the molecular representation in cell populations;
the calculation formula for the degree of cell heterogeneity is as follows:

$$\text{the degree of cell heterogeneity} = \frac{200}{\pi} \tan^{-1}\left( \frac{1}{m} \sum_{i=1}^{m} \sqrt{\sum_{j=1}^{n} (X_{ij} - \mu_j)^2 + 1} \right)$$

wherein, $X_{ij}$ represents the single-cell expression value of the j-th molecular representation in the i-th cell, n represents the number of molecular representations, m represents the cell number in cell populations, and $\mu_j$ represents the mean expression value of the j-th molecular representation in cell populations.

8. The detection method of any one of claims 1 to 7, wherein the detection method further comprises a step of detecting any one or a combination of at least two of microbiological safety, cell markers, cell viability, and biological efficacy.

9. A detection device for a cell quality attribute, wherein the detection device comprises a detection module for cell heterogeneity, and the detection module for cell heterogeneity is configured to detect the state diversity of cell subpopulations, wherein the state diversity of cell subpopulations comprises any one or a combination of at least two of the genome diversity, transcriptome diversity, proteome diversity, metabolome diversity and epigenome diversity of cell subpopulations.

10. The detection device of claim 9, wherein the detection module for cell heterogeneity comprises a detection submodule for the degree of cell heterogeneity, comprising:

a determination unit for molecular representation, for determining a molecular representation, a single-cell expression value of the molecular representation, and a mean expression value of the molecular representation in cell populations, based on a single-cell molecular expression matrix of cell populations; and
a calculation unit, for determining a degree of cell heterogeneity based on the single-cell expression value of the molecular representation and the mean expression value of the molecular representation in cell populations.

11. The detection device of claim 9, wherein the detection module for cell heterogeneity further comprises a detection submodule for single-cell molecular expression matrix, comprising:

a detection unit for single-cell molecular expression data, for detecting a molecular expression value of cell populations at a single-cell level to obtain a single-cell molecular expression data of cell populations; and
a data preprocessing unit, for preprocessing the single-cell molecular expression data of cell populations to obtain a single-cell molecular expression matrix of cell populations; wherein the preprocessing comprises noise correction and/or standardization.

12. The detection device of claim 10, wherein the determination unit for molecular representation is used to process the single-cell molecular expression matrix by using a method of data dimensionality reduction, to select non-linearly related data and non-biological function related data as molecular representations, and to determine the single-cell expression value of the molecular representation and the mean expression value of the molecular representation in cell populations.

13. The detection device of claim 10, wherein the molecular representation is selected from any one or a combination of at least two of gene representation, transcriptional representation, protein representation, metabolite representation, and epigenetic site representation that can represent single-cell molecular expression matrix of cell populations.

14. The detection device of claim 10, wherein the calculation unit is used to calculate the Euclidean distance and determine the degree of cell heterogeneity based on the single-cell expression value of the molecular representation and the mean expression value of the molecular representation in cell populations.

15. The detection device of claim 10, wherein the calculation unit is used to determine the degree of cell heterogeneity based on the calculation formula of the degree of cell heterogeneity according to the single cell expression value of the molecular representation and the mean expression value of the molecular representation in cell populations; the calculation formula for the degree of cell heterogeneity is as follows:

$$\text{the degree of cell heterogeneity} = \frac{200}{\pi} \tan^{-1}\left( \frac{1}{m} \sum_{i=1}^{m} \sqrt{\sum_{j=1}^{n} (X_{ij} - \mu_j)^2 + 1} \right)$$

wherein, $X_{ij}$ represents the single-cell expression value of the j-th molecular representation in the i-th cell, n represents the number of molecular representations, m represents the cell number in cell populations, and $\mu_j$ represents the mean expression value of the j-th molecular representation in cell populations.

16. The detection device of claim 9, wherein the detection device further comprises:
any one or a combination of at least two of a detection module for microbiological safety, a detection module for cell marker, a detection module for cell viability, and a detection module for biological efficacy.

17. Use of the detection method of a cell quality attribute of any one of claims 1 to 8 and/or the detection device for a cell quality attribute of any one of claims 9 to 16 in the manufacture of cell products.

18. The use of claim 17, wherein the cell product comprises a living mammalian cell that has been or has not been genetically modified, and optionally, any one or a combination of at least two of an adjuvant, a carrier, an excipient, and a diluent, which are acceptable pharmaceutically; wherein the mammal comprises a human.

19. The use of claim 17, wherein the cell product comprises any one or a combination of at least two of autologous or allogeneic stem cell products, immune cell products, tissue cell products, and cell line products.

20. The use of claim 19, wherein the stem cell comprises any one or a combination of at least two of an adult stem cell, an embryonic stem cell, an induced pluripotent stem cell, a stem cell derived from the transformation of a mature somatic cell, and cells derived therefrom.

21. The use of claim 19, wherein the stem cell comprises any one or a combination of at least two of a mesenchymal stem cell, a mesenchymal stromal cell, a multipotent stromal cell, a multipotent mesenchymal stromal cell, and a drug signaling cell.

22. The use of claim 19, wherein the stem cell comprises any one or a combination of at least two of an adipose-derived stem cell, an umbilical cord-derived stem cell, a placenta-derived stem cell, a bone marrow-derived stem cell, a dental pulp-derived stem cell, a uterine blood-derived stem cell, an amniotic epithelial stem cell, and a bronchial basal layer cell.

23. Use of the detection method of a cell quality attribute of any one of claims 1 to 8 and/or the detection device for a cell quality attribute of any one of claims 9 to 16 in the research and/or development of cell products.

24. The use of claim 23, wherein the cell product comprises a cell stock solution product and/or a cell preparation product; the cell stock solution product comprises a cell seed bank product and/or a cell working bank product.

25. The use of claim 23, wherein the research and/or development of cell products comprises the research and/or development of any one or a combination of at least two of a preparation process of cell seed bank products, storage stability of cell seed bank products, a preparation process of cell working bank products, storage stability of cell working bank products, a preparation process of cell preparation products and stability of cell preparation products.

Figure 1

| Detection module for cell heterogeneity | |
| detect the state diversity of cell subpopulations (genome diversity, transcriptome diversity, proteome diversity, metabolome diversity and epigenome diversity of cell subpopulations) | 10 |

| Detection module for microbiological safety | |
| detect mycoplasma, bacteria, fungi, endotoxins, mycobacteria and the like in cell samples | 20 |

| Detection module for cell marker | |
| detect cell surface markers (such as CD73, CD90, CD105, CD11b, CD19, CD34, CD45 and HLA-DR) | 30 |

| Detection module for cell viability | |
| detect cell viability, cell doubling time, cell cycle, cell apoptosis, clone formation rate and the like | 40 |

| Detection module for biological efficacy | |
| detect the ability of osteogenic differentiation, adipogenic differentiation, chondrogenic differentiation and the like | 50 |

Figure 2A

**Detection submodule for the matrix of single cell molecular expression**
detect the matrix of single cell molecular expression in cell populations — 110

**Detection submodule for the degree of cell heterogeneity**
detect the degree of cell heterogeneity in cell populations — 120

Figure 2B

**Detection unit for single-cell molecular expression data**
detect the molecular expression value of cell populations at a single-cell level and obtaining single-cell molecular expression data of cell populations — 1101

**Data preprocessing unit**
detect the single-cell molecular expression data of cell populations and obtaining a single-cell molecular expression matrix of cell populations — 1102

Figure 2C

**Determination unit for molecular representation**
for processing the single-cell molecular expression matrix by using a method of data dimensionality reduction, selecting non-linearly related data and non-biological function related data as molecular representations, and determining the single-cell expression value of the molecular representation and the mean expression value of the molecular representation in cell populations — 1201

**Calculation unit**
for determining the degree of cell heterogeneity based on the calculation formula for the degree of cell heterogeneity according to the single cell expression value of the molecular representation and the mean expression value of the molecular representation in cell populations — 1202

Figure 2D

Figure 3

Figure 4A

Figure 4B

Figure 4C

Figure 5A

Figure 5B

Figure 6A

Figure 6B

Figure 6C

Figure 6D

Figure 7A

Figure 7B

Figure 7C

Figure 8A

Figure 8B

Figure 8C

Figure 9

Figure 10

Figure 11A

Figure 11B

Figure 11C

Figure 11D

Figure 11E

Figure 11F

0-6 hours

Mean value: 77.83

95%CI: 75.76~79.90

99%CI: 74.97~80.69

The degree of cell heterogeneity

Figure 12A

6-24 hours

Mean value: 72.34

95%CI: 66.42~78.26

99%CI: 64.16~80.53

The degree of cell heterogeneity

Figure 12B

0-24 hours

Mean value: 75.09

95%CI: 71.97~78.20

99%CI: 70.89~79.28

The degree of cell heterogeneity

Figure 12A                    Figure 12B                    Figure 12C

## INTERNATIONAL SEARCH REPORT

| International application No. |
| --- |
| **PCT/CN2023/141519** |

**A. CLASSIFICATION OF SUBJECT MATTER**

G16B 30/00(2019.01)i

According to International Patent Classification (IPC) or to both national classification and IPC

**B. FIELDS SEARCHED**

Minimum documentation searched (classification system followed by classification symbols)

IPC: G16B

Documentation searched other than minimum documentation to the extent that such documents are included in the fields searched

Electronic data base consulted during the international search (name of data base and, where practicable, search terms used)

CNTXT, WPABSC, ENTXTC, ENTXT, USTXT, WOTXT, CJFD, VEN, CNKI, DWPI, 万方, WANFANG, 百度, BAIDU, bing, PUBMED, ISI Web of Knowledg, 读秀, DUXIU: 细胞, 异质性, 基因组, 转录组, 蛋白质组, 代谢组, 表观组, 多样性, 单细胞, 表达, 基因, 蛋白, 矩阵, 噪音, 过滤, 校正, 标准化, 预处理, 降维, 欧式距离, 装置, 设备, 元件, Cell, heterogeneity, genome, transcriptome, proteome, metabolome, epime, diversity, single cell, express+, gene, protein, matrix, noise, filter+, correct+, normaliz+, preprocess+, dimensionality reduction, Euclidean distance, device, element.

**C. DOCUMENTS CONSIDERED TO BE RELEVANT**

| Category* | Citation of document, with indication, where appropriate, of the relevant passages | Relevant to claim No. |
| --- | --- | --- |
| X | CN 112735536 A (HUNAN UNIVERSITY) 30 April 2021 (2021-04-30) description, paragraphs 2-3 and 8-43 | 1, 9, 23 |
| Y | CN 112735536 A (HUNAN UNIVERSITY) 30 April 2021 (2021-04-30) description, paragraphs 2-3 and 8-43 | 2-6, 8, 10-14, 16-25 |
| X | CN 109979538 A (GUANGZHOU GENE DENOVO BIOTECHNOLOGY CO., LTD.) 05 July 2019 (2019-07-05) description, paragraphs 69-112 | 1 |
| Y | CN 109979538 A (GUANGZHOU GENE DENOVO BIOTECHNOLOGY CO., LTD.) 05 July 2019 (2019-07-05) description, paragraphs 69-112 | 2-6, 8, 10-14, 16-25 |
| A | CN 113470743 A (HARBIN NEBULA MEDICAL LABORATORY CO., LTD.) 01 October 2021 (2021-10-01) entire document | 1-25 |

☑ Further documents are listed in the continuation of Box C.    ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
| --- | --- |
| **27 March 2024** | **01 April 2024** |

| Name and mailing address of the ISA/CN | Authorized officer |
| --- | --- |
| **China National Intellectual Property Administration (ISA/CN)** **China No. 6, Xitucheng Road, Jimenqiao, Haidian District, Beijing 100088** | |
| | Telephone No. |

Form PCT/ISA/210 (second sheet) (July 2022)

## INTERNATIONAL SEARCH REPORT

<table>
<tr><td colspan="2"></td><td>International application No.</td></tr>
<tr><td colspan="2"></td><td>**PCT/CN2023/141519**</td></tr>
</table>

**C.    DOCUMENTS CONSIDERED TO BE RELEVANT**

| Category* | Citation of document, with indication, where appropriate, of the relevant passages | Relevant to claim No. |
|---|---|---|
| A | WO 2005081635 A2 (YEDA RESEARCH AND DEVELOPMENT CO., LTD.) 09 September 2005 (2005-09-09)<br>    entire document | 1-25 |
| A | GAN, Yanglan et al. "Identification of Cancer Subtypes from Single-Cell RNA-Seq Data Using a Consensus Clustering Method"<br>*BMC Medical Genomics*, No. 11 (Suppl 6), 31 December 2018 (2018-12-31),<br>    Article 117: pages 66-72 | 1-25 |
| A | KISELEV, V. Y. et al. "SC 3: consensus clustering of single-cell RNARNARNA-seq data"<br>*Nature Methods*, Vol. 14, No. 5, 27 March 2017 (2017-03-27),<br>    pages 483-486 | 1-25 |

**INTERNATIONAL SEARCH REPORT**
Information on patent family members

International application No.

**PCT/CN2023/141519**

| Patent document cited in search report | | | Publication date (day/month/year) | Patent family member(s) | | | Publication date (day/month/year) |
|---|---|---|---|---|---|---|---|
| CN | 112735536 | A | 30 April 2021 | None | | | |
| CN | 109979538 | A | 05 July 2019 | None | | | |
| CN | 113470743 | A | 01 October 2021 | None | | | |
| WO | 2005081635 | A2 | 09 September 2005 | GB | 0618126 | D0 | 25 October 2006 |
| | | | | GB | 2427728 | A | 03 January 2007 |
| | | | | GB | 2427728 | B | 20 August 2008 |
| | | | | WO | 2005081635 | A3 | 04 January 2007 |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202211677045

**Non-patent literature cited in the description**

- **KUMAR RM** ; **CAHAN P** ; **SHALEK AK** ; **SATIJA R** ; **DALEYKEYSER A** ; **LI H, ZHANG J** ; **PARDEE K** ; **GENNERT D** ; **TROMBETTA JJ** ; **FERRANTE TC**. Deconstructing transcriptional heterogeneity in pluripotent stem cells. *Nature*, 04 December 2014, vol. 516 (7529), 56-61

- **M. DOMINICI et al.** Minimal criteria for defining multipotent mesenchymal stromal cells. The International Society for Cellular Therapy position statement. *Cytotherapy*, 2006, vol. 8 (4), 315-317
- **HUANG et al.** SAVER: gene expression recovery for single-cell RNA sequencing. *Nature Methods*, 2018, vol. 15, 539-542